(19) Europäisches Patentamt
European Patent Office
Office européen des brevets

(11) Veröffentlichungsnummer : **0 297 345 B2**

# (12) NEUE EUROPÄISCHE PATENTSCHRIFT

(45) Veröffentlichungstag der neuen Patentschrift : **13.10.93 Patentblatt 93/41**

(21) Anmeldenummer : **88109424.7**

(22) Anmeldetag : **14.06.88**

(51) Int. Cl.[5] : **C07D 249/08,** C07D 233/60, A01N 43/653, C07C 33/34, C07C 29/40, C07D 303/08

## (54) Azolylmethyl-cyclopropyl-Derivate.

Die Akte enthält technische Angaben, die nach dem Eingang der Anmeldung eingereicht wurden und die nicht in dieser Patentschrift enthalten sind.

(30) Priorität : **24.06.87 DE 3720755**
**19.04.88 DE 3812967**

(43) Veröffentlichungstag der Anmeldung : **04.01.89 Patentblatt 89/01**

(45) Bekanntmachung des Hinweises auf die Patenterteilung : **25.09.91 Patentblatt 91/39**

(45) Bekanntmachung des Hinweises auf die Entscheidung über den Einspruch : **13.10.93 Patentblatt 93/41**

(84) Benannte Vertragsstaaten : **AT BE CH DE ES FR GB GR IT LI NL SE**

(56) Entgegenhaltungen :
**EP-A- 0 015 756**
**EP-A- 0 044 993**
**EP-A- 0 047 594**
**EP-A- 0 072 580**
**EP-A- 0 080 102**
**EP-A- 0 091 398**
**EP-A- 0 139 227**
**EP-A- 0 180 136**

(73) Patentinhaber : **BAYER AG**
**D-51368 Leverkusen (DE)**

(72) Erfinder : **Stroech, Klaus, Dr.**
**Rolsberger Strasse 22**
**D-5650 Solingen 19 (DE)**
Erfinder : **Brandes, Wilhelm, Dr.**
**Eichendorffstrasse 3**
**D-5653 Leichlingen (DE)**
Erfinder : **Dutzmann, Stefan, Dr.**
**Leinenweberweg 33**
**D-4000 Düsseldorf 13 (DE)**

EP 0 297 345 B2

**Beschreibung**

Die vorliegende Erfindung betrifft neue Azolylmethylcyclopropyl-Derivate, mehrere Verfahren zu deren Herstellung und deren Verwendung als Fungizide und Pflanzenwachstumsregulatoren.

Es ist bereits bekannt, daß bestimmte Azolylmethylcyclopropyl-carbinol-Derivate fungizide und pflanzenwuchsregulierende Eigenschaften besitzen (vgl. EP-A 0 180 136). So können z.B. 1-(4-Chlorphenyl)-1-(1-chlorcyclopropyl)-2-(1,2,4-triazol-1-yl)-ethan-1-ol und 1-(4-Fluorphenyl)-(1-chlor-cyclopropyl)-2-(1,2,4-triazol-1-yl)-ethan-1-ol zur Bekämpfung von Pilzen und zur Regulierung des Pflanzenwachstums eingesetzt werden. Die Wirksamkeit dieser Stoffe ist gut; sie läßt allerdings bei niedrigen Aufwandmengen in manchen Fällen zu wünschen übrig.

Weiterhin ist bekannt, daß bestimmte durch Cycloalkyl substituierte Hydroxyethyl-azolyl-Derivate fungizide Eigenschaften besitzen (vgl. EP- A 0 015 756). Auch die Wirksamkeit dieser Stoffe ist aber nicht immer voll ausreichend.

Aus der EP-A 0 047 594 sind Hydroxyethyl-triazolyl-Derivate mit fungiziden und pflanzenwuchsregulierenden Eigenschaften bekannt. Es werden aber keine Verbindungen beschrieben in denen das Carbinol-Kohlenstoffatom mit einem substituierten Cyclopropylrest verbunden ist. Schließlich werden in der EP-A 0 072 580 fungizid und pflanzenwuchsregulierend wirksame Hydroxyethyl-azolyl-Derivate offenbart in denen einer der Reste am zentralen Kohlenstoffatom für gegebenenfalls durch Methyl substituiertes Cyclopropyl stehen kann. Entsprechende Stoffe, in denen zusätzlich ein gegebenenfalls substituierter Benzyl-Substituent vorhanden ist, werden aber nicht erwähnt.

Es wurden nun neue Azolylmethyl-cyclopropyl-Derivate der Formel

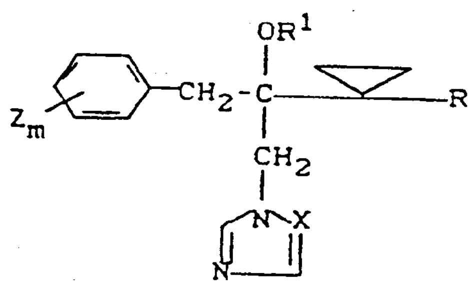

(I)

in welcher

R für Fluor, Chlor, Brom, Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen. substituiertes Phenyl oder für die Gruppierung -Y-$R^2$ steht, worin

Y für Sauerstoff, Schwefel, SO oder $SO_2$ steht und

$R^2$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,

$R^1$ für Wasserstoff, Alkyl mit 1 bis 6 Kohlenstoffatomen oder Alkylcarbonyl mit 1 bis 6 Kohlenstoffatomen in der Alkylgruppe steht,

X für Stickstoff oder eine CH-Gruppe steht,

Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht und

m für die Zahlen 0, 1, 2 oder 3 steht,

sowie deren Säureadditions-Salze und Metallsalz-Komplexe gefunden.

Weiterhin wurde gefunden, daß man Azolylmethyl-cyclopropyl-Derivate der Formel (I) sowie deren Säureadditions-Salze und Metallsalz-Komplexe erhält, wenn man

a) Propanol-Derivate der Formel

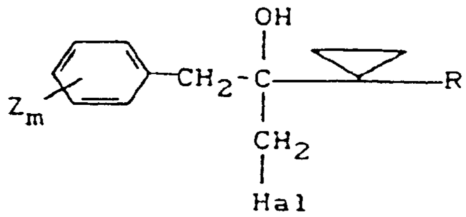

(II)

in welcher
R, Z und m die oben angegebene Bedeutung haben und Hal für Chlor, Brom oder Jod steht,
mit Azolen der Formel

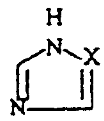

(III)

in welcher
X die oben angegebene Bedeutung hat,
in Gegenwart eines Säurebindemittels und in Gegenwart eines Verdünnungsmittels umsetzt, oder
b) Oxirane der Formel

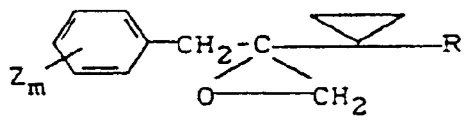

(IV)

in welcher
R, Z und m die oben angegebene Bedeutung haben,
mit Azolen der Formel

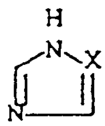

(III)

in welcher
X die oben angegebene Bedeutung hat,
in Gegenwart eines Säurebindemittels und in Gegenwart eines Verdünnungsmittels umsetzt.
oder
c) Azolylmethyl-ketone der Formel

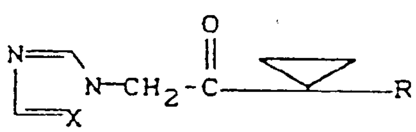

(V)

in welcher
R und X die oben angegebene Bedeutung haben,
mit metallorganischen Verbindungen der Formel

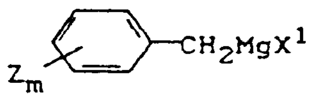

(VI)

in welcher
Z und m die oben angegebene Bedeutung haben und
$X^1$ für Chlor, Brom oder Iod steht,
in Gegenwart eines Verdünnungsmittels umsetzt, oder
d) Azolylmethyl-cyclopropyl-carbinol-Derivate der Formel

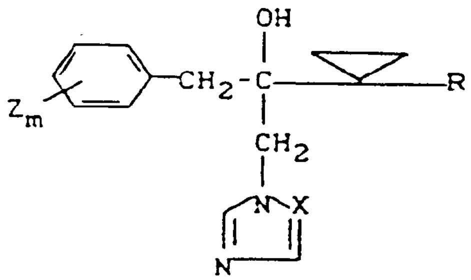

(Ia)

in welcher
R, X, Z und m die oben angegebene Bedeutung haben,
mit starken Basen in Gegenwart eines Verdünnungsmittels umsetzt und die dabei entstehenden Alkoholate der Formel

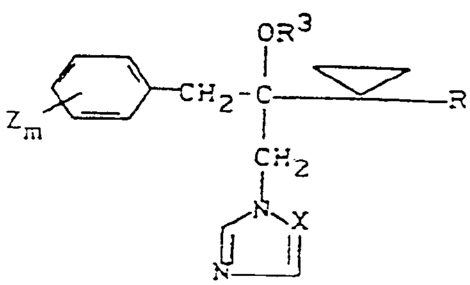

(Ib)

in welcher
R, X, Z und m die oben angegebene Bedeutung haben und
$R^3$ für ein Alkalimetallkation oder für ein quarternäres Ammonium- oder Phosphoniumkation steht,
mit Halogenverbindungen der Formel

$R^4$-Hal' (VII)

in welcher
$R^4$ für Alkyl mit 1 bis 6 Kohlenstoffatomen oder Alkylcarbonyl mit 1 bis 6 Kohlenstoffatomen im Alkylteil steht und
Hal' für Chlor oder Brom steht, in Gegenwart eines Verdünnungsmittels umsetzt,
und gegebenenfalls anschließend an die so erhaltenen Verbindungen der Formel (I) eine Säure oder ein Metallsalz addiert.

Schließlich wurde gefunden, daß die neuen Azolylmethylcyclopropyl-Derivate der Formel (I) sowie deren Säure-additions-Salze und Metallsalz-Komplexe starke fungizide und pflanzenwuchsregulierende Eigenschaften besitzen.

Die erfindungsgemäßen Stoffe enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische

Überraschenderweise besitzen die erfindungsgemäßen Stoffe eine bessere fungizide und pflanzenwuchsregulierende Wirksamkeit als die konstitutionell ähnlichsten vorbekannten Verbindungen gleicher Wirkungsrichtung.

Die erfindungsgemäßen Azolylmethyl-cyclopropyl-Derivate sind durch die Formel (I) allgemein definiert.

Bevorzugt sind diejenigen Verbindungen der Formel (I), in denen

R für Fluor, Chor, Brom, Methyl, Ethyl, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung -Y-$R^2$ steht, worin

Y für Sauerstoff, Schwefel, SO oder $SO_2$ steht und

$R^2$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht,

$R^1$ für Wasserstoff, Methyl, Ethyl, n-Propyl, Isopropyl, n-Butyl, Isobutyl, Methylcarbonyl, Ethylcarbonyl, n-Propylcarbonyl, Isopropyl-carbonyl, n-Butylcarbonyl und Isobutyl-carbonyl steht,

X für Stickstoff oder eine CH-Gruppe steht.

Z für Fluor, Chlor, Brom Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht und

m für die Zahlen 0, 1, 2 oder 3 steht.

Bevorzugte erfindungsgemäße Verbindungen sind auch Additionsprodukte aus Säuren und denjenigen Azolylmethylcyclopropyl-Derivaten der Formel (I), in denen R, $R^1$, X, Z und m die Bedeutungen haben, die bereits vorzugsweise für diese Reste bzw. diesen Index genannt wurden

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der Elemente und denjenigen Azolylmethyl-cyclopropyl-Derivaten der Formel (I), in denen R, $R^1$, X, Z und m die Bedeutungen haben, die bereits vorzugsweise für diese Reste und diesen Index genannt wurden. Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für Azolylmethyl-cyclopropyl-Derivate der Formel (I) seien die in der folgenden Tabelle aufgeführten Stoffe genannt.

Tabelle

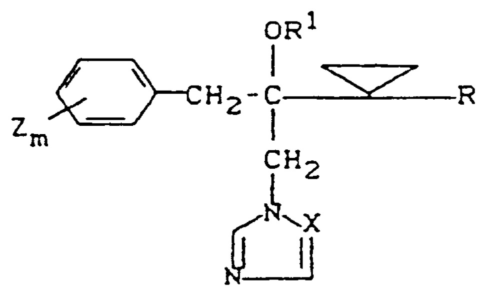

(I)

| $Z_m$ | $R^1$ | X | R |
|---|---|---|---|
| 4-Cl | H | N | Cl |
| 2,4-$Cl_2$ | " | " | Cl |
| 2,4-$F_2$ | " | " | Cl |
| 4-$CH_3$ | " | " | Cl |
| 4-$CF_3$ | " | " | Cl |
| 4-$OCF_3$ | " | " | Cl |
| 4-$OCH_3$ | " | " | Cl |
| 4-$SCH_3$ | " | " | Cl |

Tabelle (Fortsetzung)

| $Z_m$ | $R^1$ | X | R |
|---|---|---|---|
| 2,4,6-$Cl_3$ | " | " | Cl |
| 4-Cl | " | " | F |
| 4-Cl | " | CH | Cl |
| 4-Cl | $CH_3$ | N | Cl |
| 4-Cl | H | " | 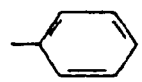 |
| 4-Cl | " | " | 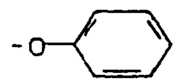 |
| 4-Cl | " | " | 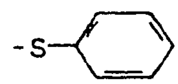 |
| 4-Cl | " | " | 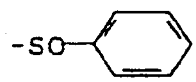 |
| 4-Cl | " | " | 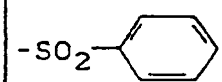 |
| 4 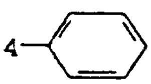 | " | " | Cl |
| 4- 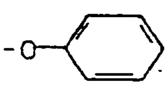 | " | " | Cl |
| 4-$C_4H_9$-t. | " | " | Cl |
| 2-Cl, 4-$CH_3$ | " | " | Cl |
| - | " | " | Cl |
| 4-Cl | -CO-$CH_3$ | " | Cl |
| 4-Cl | -$C_2H_5$ | " | Cl |
| 4-F | $CH_3$ | " | F |
| 4-Cl | H | " | $CH_3$ |

Verwendet man 1-Chlor-2-(1-chlorcyclopropyl)-3-(4-fluorphenyl)-propan-2-ol und 1,2,4-Triazol als Ausgangsstoffe, so kann der Verlauf des erfindungsgemäßen Verfahrens (a) durch das folgende Formelschema veranschaulicht werden:

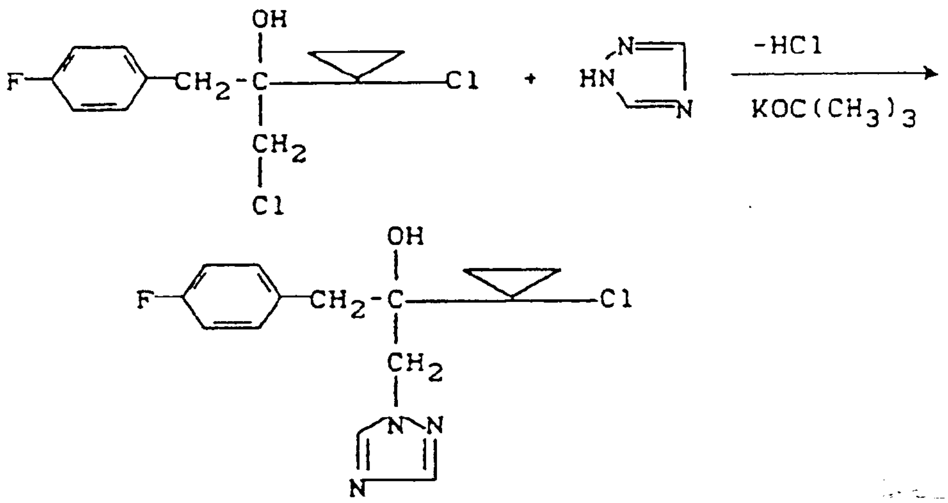


Verwendet man 2-[(4-Fluorphenyl)-methyl]-2-(1-chlorcyclopropyl)-oxiran und 1,2,4-Triazol als Ausgangsstoffe, so kann der Verlauf des erfindungsgemäßen Verfahrens (b) durch das folgende Formelschema veranschaulicht werden:

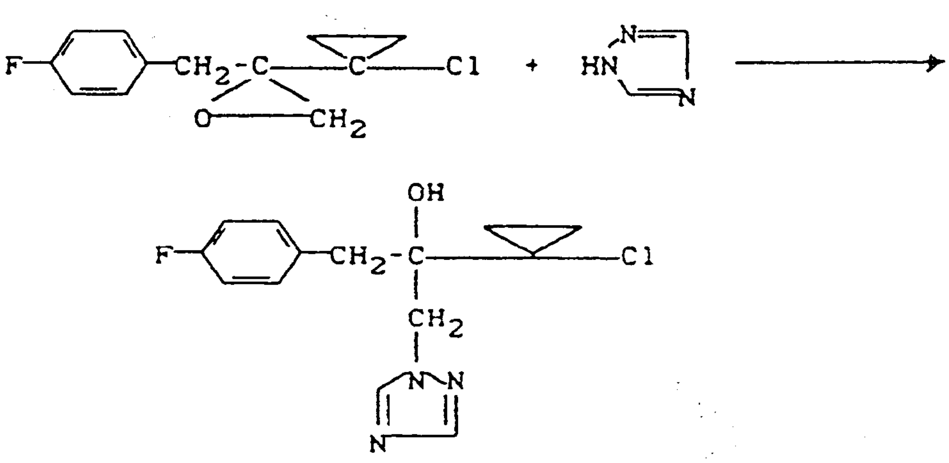

Verwendet man (1,2,4-Triazol-1-yl-methyl)-(1-chlorcycloprop-1-yl)-keton und 4-Fluorbenzyl-magnesium-bromid als Ausgangsstoffe, so kann der Verlauf des erfindungsgemäßen Verfahrens (c) durch das folgende Formelschema veranschaulicht werden:

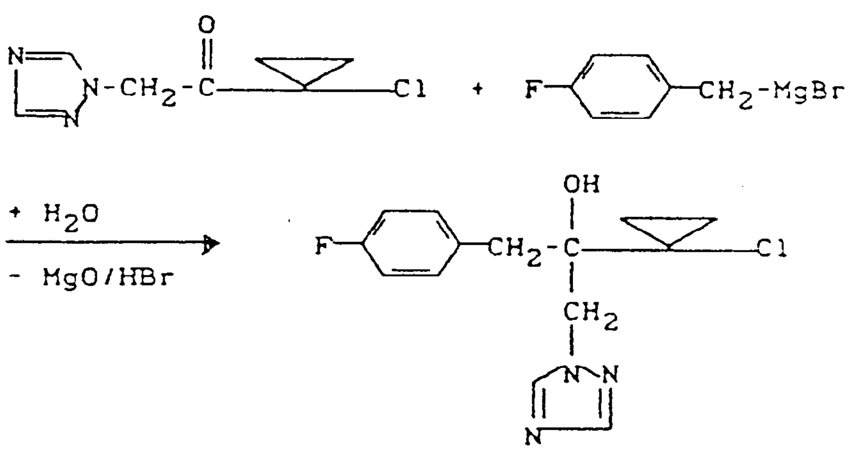


Verwendet man 1-(4-Fluorphenyl)-2-(1-chlorcyclopropyl)-3-(1,2,4-triazol-1-yl)-propan-2-ol und Natriumhydrid

als Ausgangsstoffe und Iodmethan als Reaktionskomponente, so kann der Verlauf des erfindungsgemäßen Verfahrens (d) durch das folgende Formelschema veranschaulicht werden:

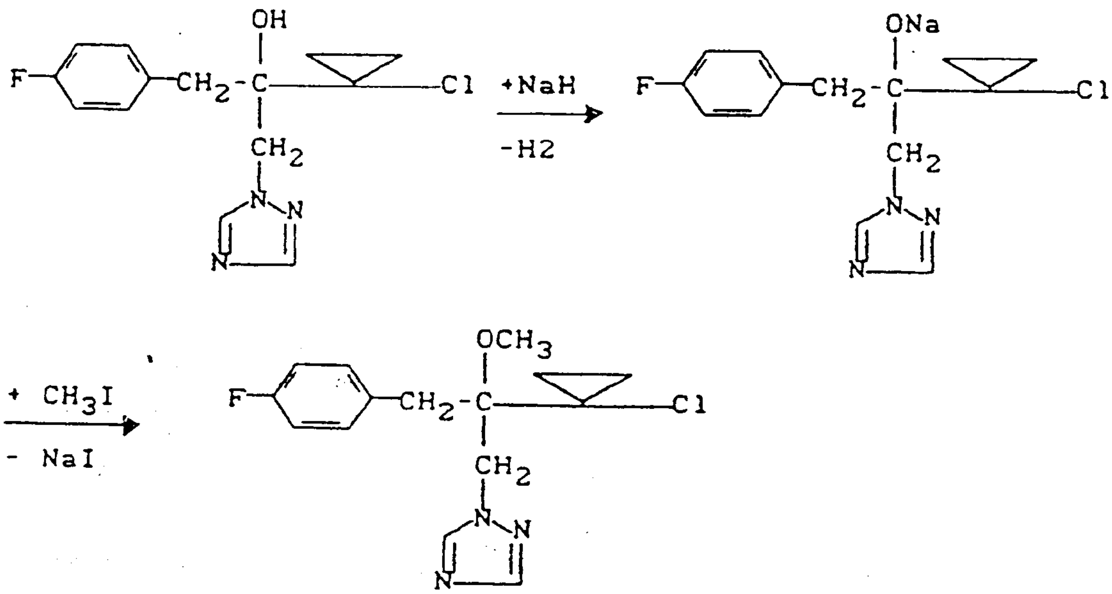


Die bei dem erfindungsgemäßen Verfahren (a) als Ausgangsstoffe benötigten Propanol-Derivate sind durch die Formel (II) allgemein definiert. In dieser Formel haben R, Z und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden. Hal steht vorzugsweise für Chlor oder Brom.

Die Propanol-Derivate der Formel (II) sind bisher noch nicht bekannt, Sie lassen sich herstellen, indem man Cyclopropylketone der Formel

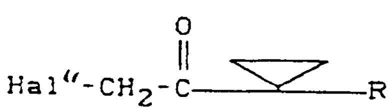
(VIII)

in welcher
R die oben angegebene Bedeutung hat und
Hal" für Chlor oder Brom steht,
mit metallorganischen Verbindungen der Formel

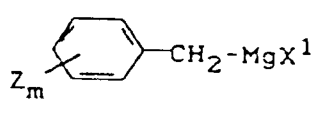
(VI)

in welcher
$X^1$, Z und m die oben angegebene Bedeutung haben,
in Gegenwart eines Verdünnungsmittels umsetzt.

Die bei der Herstellung der Propanol-Derivate nach dem obigen Verfahren als Ausgangsstoffe benötigten Cyclopropylketone der Formel (VIII) sind teilweise bekannt. Sie lassen sich herstellen, indem man Ketone der Formel

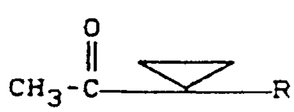
(IX)

in welcher
R die oben angegebene Bedeutung hat,
mit Chlorierungsmitteln oder Bromierungsmitteln in Gegenwart eines Verdünnungsmittels umsetzt.

Die bei der Herstellung von Cyclopropylketonen der Formel (VIII) als Ausgangsstoffe benötigten Ketone der Formel (IX) sind bekannt oder lassen sich nach prinzipiell bekannten Verfahren synthetisieren (vgl. Synthesis 1977, 189).

Als Chlorierungsmittel und Bromierungsmittel kommen bei dem obigen Verfahren zur Herstellung von Cyclopropylketonen der Formel (VIII) alle für derartige Umsetzungen üblichen Chlorierungs- und Bromierungs-Reagenzien in Betracht. Vorzugsweise verwendbar sind Sulfurylchlorid, Sulfurylbromid und Brom.

Als Verdünnungsmittel kommen bei der Herstellung von Cyclopropylketonen der Formel (VIII) nach dem obigen Verfahren alle für derartige Umsetzungen üblichen inerten organischen Solventien infrage. Vorzugsweise verwendbar sind halogenierte aliphatische Kohlenwasserstoffe, wie Methylenchlorid, Chloroform und Tetrachlorkohlenstoff.

Die Temperaturen können bei dem obigen Verfahren zur Herstellung von Cyclopropylketonen der Formel (VIII) innerhalb eines bestimmten Bereiches variiert werden. Im allgemeinen arbeitet man bei Temperaturen zwischen -10°C und 60°C, vorzugsweise zwischen 0°C und +40°C.

Bei der Durchführung des obigen Verfahrens zur Herstellung von Cylcopropylketonen der Formel (VIII) arbeitet man im allgemeinen unter Normaldruck. Es ist jedoch auch möglich, unter erhöhtem oder vermindertem Druck zu arbeiten.

Bei der Durchführung des obigen Verfahrens zur Herstellung von Cyclopropylketonen der Formel (VIII) setzt man auf 1 Mol an Keton der Formel (IX) im allgemeinen eine stöchiometrische Menge oder auch einen geringen Überschuß an Chlorierungs- oder Bromierungsmittel ein. Die Aufarbeitung erfolgt nach üblichen Methoden. Im allgemeinen geht man so vor, daß man das Reaktionsgemisch nacheinander mit verdünnter, wäßriger Natriumhydrogencarbonat-Lösung und mit Wasser wäscht, dann trocknet und einengt.

Die bei dem obigen Verfahren zur Herstellung von Propanol-Derivaten der Formel (II) als Reaktionskomponenten benötigten metallorganischen Verbindungen der Formel (VI) sind bekannt oder lassen sich nach prinzipiell bekannten Methoden synthetisieren. So erhält man Verbindungen der Formel (VI), indem man Benzylhalogenide der Formel

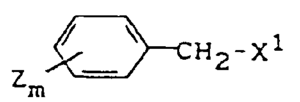

(X)

in welcher
$X^1$, Z und m die oben angegebene Bedeutung haben,
mit Magnesium in Gegenwart eines inerten Verdünnungsmittels, wie z.B. Diethylether, bei Temperaturen zwischen 0°C und 50°C umsetzt.

Die Benzylhalogenide der Formel (X) sind allgemein bekannte Verbindungen der organischen Chemie.

Als Verdünnungsmittel kommen bei dem obigen Verfahren zur Herstellung von Propanol-Derivaten der Formel (II) alle für derartige Umsetzungen üblichen inerten organischen Solventien in Betracht. Vorzugsweise verwendbar sind Ether, wie Diethylether, Tetrahydrofuran und Dioxan.

Die Reaktionstemperaturen können bei der Durchführung des obigen Verfahrens zur Herstellung von Propanol-Derivaten der Formel (II) innerhalb eines bestimmten Bereiches variiert werden. Im allgemeinen arbeitet man bei Temperaturen zwischen -80° C und + 50° C, vorzugsweise zwischen -80°C und 40°C.

Bei der Durchführung des obigen Verfahrens zur Herstellung von Propanol-Derivaten der Formel (II) arbeitet man im allgemeinen unter Normaldruck. Es ist jedoch auch möglich, unter erhöhtem oder vermindertem Druck zu arbeiten.

Bei der Durchführung des obigen Verfahrens zur Herstellung von Propanol-Derivaten der Formel (II) setzt man auf 1 Mol an Cyclopropylketon der Formel (VIII) im allgemeinen 1 bis 1,2 Mol an metallorganischer Verbindung der Formel (VI) ein, die zweckmäßigerweise unmittelbar zuvor hergestellt und in situ weiterverarbeitet wird. Die Aufarbeitung erfolgt nach üblichen Methoden. Im allgemeinen geht man so vor, daß man zunächst ansäuert und mit Wasser versetzt, dann die organische Phase abtrennt. wäscht und nach dem Trocknen einengt.

Die bei der Durchführung des erfindungsgemäßen Verfahrens (a) als Reaktionskomponenten benötigten Azole der Formel (III) sind allgemein bekannte Verbindungen der organischen Chemie.

Als Säurebindemittel kommen bei der Durchführung des erfindungsgemäßen Verfahrens (a) alle üblichen anorganischen und organischen Basen infrage. Vorzugsweise verwendbar sind Alkalicarbonate, wie Natrium-und Kaliumcarbonat, ferner Alkalimetallhydroxide, wie Natrium- und Kaliumhydroxid, außerdem Alkalimetallalkoholate, wie Natrium- und Kaliummethylat und -ethylat sowie Kalium-tert.-butylat, und weiterhin niedere tertiäre Alkylamine, Cycloalkylamine und Aralkylamine, wie insbesondere Triethylamin.

Als Verdünnungsmittel kommen bei der Durchführung des erfindungsgemäßen Verfahrens (a) alle üblichen inerten organischen Solventien in Betracht. Vorzugsweise verwendbar sind Nitrile, wie Acetonitril, ferner aromatische Kohlenwasserstoffe, wie Benzol, Toluol und Dichlorbenzol, außerdem Formamide, wie Dimethylformamid, sowie stark polare Lösungsmittel, wie Dimethylsulfoxid und Hexamethylphosphorsäuretriamid.

Die Reaktionstemperaturen können bei der Durchführung des erfindungsgemäßen Verfahrens (a) innerhalb eines größeren Bereiches variiert werden. Im allgemeinen arbeitet man bei Temperaturen zwischen 0°C und 200°C, vorzugsweise zwischen 60°C und 150°C.

Das erfindungsgemäße Verfahren (a) wird ebenso wie die erfindungsgemäßen Verfahren (b), (c) und (d) im allgemeinen unter Normaldruck durchgeführt. Es ist jedoch jeweils auch möglich, unter erhöhtem oder vermindertem Druck zu arbeiten.

Bei der Durchführung des erfindungsgemäßen Verfahrens (a) setzt man auf 1 Mol an Propanol-Derivat der Formel (II) im allgemeinen 1 bis 4 Mol Azol der Formel (III) sowie 1 bis 3 Mol an Säurebindemittel ein. In manchen Fällen ist es zweckmäßig, unter Schutzgasatmosphäre zu arbeiten. Die Aufarbeitung erfolgt nach üblichen Methoden. Im allgemeinen geht man so vor, daß man das Reaktionsgemisch durch Abziehen des Verdünnungsmittels einengt, den verbleibenden Rückstand in einem mit Wasser wenig mischbaren organischen Lösungsmittel aufnimmt, die organische Phase wäscht und nach dem Trocknen einengt. Das verbleibende Produkt kann gegebenenfalls weiteren Reinigungsverfahren unterzogen werden.

Die bei dem erfindungsgemäßen Verfahren (b) als Ausgangsstoffe benötigten Oxirane sind durch die Formel (IV) allgemein definiert. In dieser Formel haben R, Z und m vorzugsweise diejenigen Bedeutungen, die bereits im Zusammenhang mit der Beschreibung der erfindungsgemäßen Stoffe der Formel (I) vorzugsweise für diese Reste bzw. diesen Index genannt wurden.

Die Oxirane der Formel (IV) sind bisher noch nicht bekannt. Sie lassen sich herstellen, indem man Propanol-Derivate der Formel

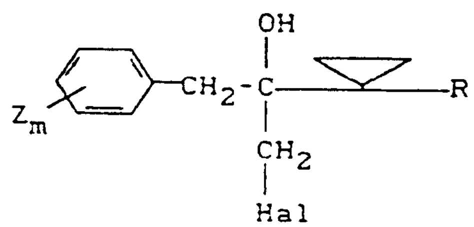

(II)

in welcher
R, Z, Hal und m die oben angegebene Bedeutung haben,
mit Basen in Gegenwart eines Verdünnungsmittels umsetzt.

Als Basen kommen bei der Herstellung von Oxiranen der Formel (IV) nach dem obigen Verfahren alle üblicherweise für derartige Umsetzungen geeigneten anorganischen und organischen Basen in Betracht. Vorzugsweise verwendbar sind alle diejenigen Basen, die bereits im Zusammenhang mit der Beschreibung des erfindungsgemäßen Verfahrens (a) als bevorzugte Säurebindemittel genannt wurden.

Die Reaktionstemperaturen können bei der Herstellung von Oxiranen nach dem obigen Verfahren innerhalb eines bestimmten Bereiches variiert werden. Im allgemeinen arbeitet man bei Temperaturen zwischen 0°C und 100°C, vorzugsweise zwischen 20°C und 60°C.

Bei der Durchführung des obigen Verfahrens zur Herstellung von Oxiranen der Formel (IV) arbeitet man im allgemeinen unter Normaldruck. Es ist jedoch auch möglich, unter erhöhtem oder vermindertem Druck zu arbeiten.

Bei der Durchführung des obigen Verfahrens zur Herstellung von Oxiranen der Formel (IV) setzt man im allgemeinen auf 1 Mol an Propanol-Derivat der Formel (II) 1 bis 3 Mol an Base ein. Die Aufarbeitung erfolgt nach üblichen Methoden.

Als Säurebindemittel und als Verdünnungsmittel kommen bei der Durchführung des erfindungsgemäßen Verfahrens (b) alle üblicherweise für derartige Umsetzungen einsetzbaren Säurebindemittel und Verdünnungsmittel infrage. Vorzugsweise verwendbar sind alle diejenigen Säurebindemittel und Verdünnungsmittel, die bereits im Zusammenhang mit der Beschreibung des erfindungsgemäßen Verfahrens (a) als bevorzugte Säurebindemittel und Verdünnungsmittel genannt wurden.

Die Reaktionstemperaturen können bei der Durchführung des erfindungsgemäßen Verfahrens (b) innerhalb eines größeren Bereiches variiert werden. Im allgemeinen arbeitet man bei Temperaturen zwischen 0°C und 200°C, vorzugsweise zwischen 60°C und 150°C.

Bei der Durchführung des erfindungsgemäßen Verfahrens (b) setzt man auf 1 Mol an Oxiran der Formel (IV) im allgemeinen 1 bis 2 Mol an Azol der Formel (III) und 1 bis 2 Mol an Säurebindemittel ein. Die Aufarbeitung erfolgt nach üblichen Methoden.

Die bei dem erfindungsgemäßen Verfahren (c) als Ausgangsstoffe benötigten Azolylmethylketone sind durch die Formel (V) allgemein definiert. In dieser Formel haben R und X vorzugsweise diejenigen Bedeutungen, die bereits im Zusammenhang mit der Beschreibung der erfindungsgemäßen Stoffe der Formel (I) vorzugsweise für diese Reste genannt wurden.

Die Azolylmethyl-ketone der Formel (V) sind teilsweise bekannt (vgl. EP-A 0 044 993, EP-A 0 080 102 und EP-A 0 139 227). Neu sind die Azolylmethyl-ketone der Formel

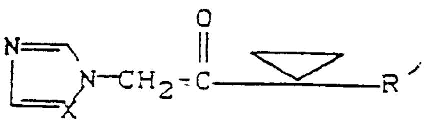

(V-a)

in welcher

R' für Fluor, Chor, Brom, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung -Y-$R^{2'}$ steht, worin

Y für Sauerstoff, Schwefel, SO oder $SO_2$ steht und

$R^{2'}$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht,
und

X für Stickstoff oder eine CH-Gruppe steht.

Sie lassen sich herstellen, indem man Cyclopropylketone der Formel

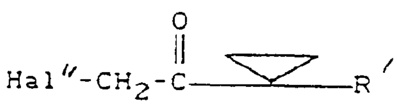

(VIII-a)

in welcher
R' und Hal" die oben angegebene Bedeutung haben,
mit Azolen der Formel

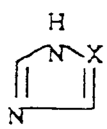

(III)

in welcher
X die oben angegebene Bedeutung hat,
in Gegenwart eines Säurebindemittels und gegebenenfalls in Gegenwart eines Verdünnungsmittels umsetzt.

Als Säurebindemittel und als Verdünnungsmittel kommen bei der Herstellung von Azolylmethyl-ketonen der Formel (V)-a nach dem obigen Verfahren alle üblicherweise für derartige Umsetzungen verwendbaren Säurebindemittel und Verdünnungsmitteiin Frage. Vorzugsweise verwendbar sind alle diejenigen Säurebindemittel, die bereits im Zusammenhang mit der Beschreibung des erfindungsgemäßen Verfahrens (a) als bevorzugte Säurebindemittel genannt wurden. Als Verdünnungsmittel kommen vorzugsweise Ketone. wie Aceton, und Nitrile, wie Acetonitril, in Betracht.

Die Reaktionstemperaturen können bei der Herstellung von Azolylmethyl-ketonen der Formel (V)-a nach dem obigen Verfahren innerhalb eines größeren Bereiches variiert werden. Im allgemeinen arbeitet man bei Temperaturen zwischen 0°C und 120°C, vorzugsweise zwischen 20°C und 100°C.

Das obige Verfahren zur Herstellung von Azolylmethylketonen der Formel (V)-a wird im allgemeinen unter Normaldruck durchgeführt.

Bei der Durchführung des obigen Verfahrens zur Herstellung von Azolylmethyl-ketonen der Formel (V)-a setzt man auf 1 Mol an Cyclopropylketon der Formel (VIII) im allgemeinen 1 bis 4 Mol an Azol der Formel (III) sowie 1 bis 3 Mol an Säurebindemittel ein. Die Aufarbeitung erfolgt nach üblichen Methoden.

Die bei dem erfindungsgemäßen Verfahren (c) als Reaktionskomponenten benötigten metallorganischen Verbindungen der Formel (VI) wurden bereits im Zusammenhang mit der Beschreibung des Verfahrens zur Herstellung der Propanol-Derivate der Formel (II) erwähnt.

Bei der Durchführung des erfindungsgemäßen Verfahrens (c) kommen als Verdünnungsmittel alle üblichen inerten organischen Solventien in Betracht. Vorzugsweise verwendbar sind Ether, wie Diethylether, Tetrahydrofuran und Dioxan.

Die Reaktionstemperaturen können auch bei der Durchführung des erfindungsgemäßen Verfahrens (c) in einem größeren Bereich variiert werden. Im allgemeinen arbeitet man bei Temperaturen zwischen -80°C und + 60°C, vorzugsweise zwischen -70°C und + 50°C.

Bei der Durchführung des erfindungsgemäßen Verfahrens (c) setzt man auf 1 Mol an Azolylmethylketon der Formel (V) im allgemeinen 0,8 bis 1 Mol an metallorganischer Verbindung der Formel (VI) ein die zweckmäßigerweise unmittelbar zuvor hergestellt und in situ weiterverarbeitet wird. Die Aufarbeitung erfolgt nach üblichen Methoden.

Die bei dem erfindungsgemäßen Verfahren (d) als Ausgangsstoffe benötigten Azolylmethylcyclopropyl-Derivate der Formel (Ia) sind erfindungsgemäße Verbindungen. Ihre Überführung in die entsprechenden Alkoholate erfolgt in allgemein bekannter Weise. indem man mit geeigneten starken Basen, wie Alkalimetallamiden oder -hydriden, quarternären Ammonium-hydroxiden oder Phosphonium-hydroxiden in einem inerten Verdünnungsmittel, wie zum Beispiel Dioxan, bei Raumtemperatur umsetzt. Demgemäß steht $R^3$ in den Verbindungen der Formel (Ib) für ein Alkalimetallkation, wie ein Natrium- oder Kaliumkation, oder für ein quarternäres Ammonium- oder Phosphoniumkation.

Die bei dem erfindungsgemäßen Verfahren (d) außerdem als Ausgangsstoffe benötigten Halogenverbindungen sind durch die Formel (VII) allgemein definiert. In dieser Formel steht $R^4$ vorzugsweise für die Bedeutungen, die bereits im Zusammenhang mit der Beschreibung der erfindungsgemäßen Stoffe der Formel (I) für den Substituenten $R^1$ genannt wurden, mit Ausnahme der Bedeutung von Wasserstoff.

Die Halogenverbindungen der Formel (VII) sind bekannt oder lassen sich nach im Prinzip bekannten Methoden herstellen.

Als Verdünnungsmittel kommen bei der Durchführung des erfindungsgemäßen Verfahrens (d) inerte organische Lösungsmittel in Frage. Hierzu gehören vorzugsweise Ether, wie Diethylether oder Dioxan; aromatische Kohlenwasserstoffe, wie Benzol; in einzelnen Fällen auch chlorierte Kohlenwasserstoffe, wie Chloroform, Methylenchlorid oder Tetrachlorkohlenstoff; sowie Hexamethylphosphorsäuretriamid.

Die Reaktionstemperaturen können bei der Durchführung des erfindungsgemäßen Verfahrens (d) in einem größeren Bereich variiert werden. Im allgemeinen arbeitet man zwischen 0°C und 120°C, vorzugsweise zwischen 20°C und 100°C.

Bei der Durchführung des erfindungsgemäßen Verfahrens (d) setzt man zunächst Hydroxy-Verbindungen der Formel (Ia) mit starken Basen zu den entsprechenden Alkoholaten der Formel (Ib) um. In der danach folgenden Stufe setzt man auf 1 Mol eines Alkoholates der Formel (Ib) vorzugsweise 1 bis 2 Mol Halogenverbindung der Formel (VII) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt. in üblicher Weise aufgearbeitet und gereinigt.

In einer bevorzugten Ausführungsform wird zweckmäßigerweise so verfahren, daß man von einer Hydroxy-Verbindung der Formel (Ia) ausgeht, letztere in einem geeigneten organischen Lösungsmittel mittels Alkalimetall-hydrid oder Alkalimetall-amid in das Alkalimetallalkoholat überführt und letzteres ohne Isolierung sofort mit einer Halogenverbindung der Formel (VII) umsetzt, wobei unter Austritt von Alkalimetallhalogenid die erfindungsgemäßen Verbindungen der Formel (I) in einem Arbeitsgang erhalten werden.

Nach einer weiteren bevorzugten Ausführungsform werden zweckmäßigerweise die Herstellung der Alkoholate sowie die Umsetzung mit einer Halogenverbindung der Formel (VII) in einem Zweiphasensystem, wie beispielsweise wäßrige Natron- oder Kalilauge/Toluol oder Methylenchlorid, unter Zusatz von 0.01-1 Mol eines Phasen-Transfer-Katalysators, wie beispielsweise Ammonium- oder Phosphoniumverbindungen, durchgeführt, wobei in der organischen Phase oder an der Grenzfläche die Alkoholate mit den in der organischen Phase befindlichen Halogeniden umgesetzt werden.

Die nach den erfindungsgemäßen Verfahren erhältlichen Azolylmethyl-cyclopropyl-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes. Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten. wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten; wie Botrytis cinerea;
Septoria-Arten, wie Septoria nodorum;
Leptosphaeria-Arten, wie Leptosphaeria nodorum;
Cercospora-Arten. wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten, wie Pseudocercosporella, sowie zur Bekämpfung von Uromyces und Botrytis im Obst-, Wein- und Gemüsebau.

Sie lassen sich mit besonders gutem Erfolg gegen Pyricularia an Reis sowie gegen Leptosphaeria nodorum, Pyrenophora teres sowie Rost und Mehltau in Getreidekulturen verwenden. Außerdem zeigen die erfindungsgemäßen Stoffe eine gute in-vitro-Wirkung.

Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb als

Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchffall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen

oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen. wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein. um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden; wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen

oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew -%, vorzugsweise zwischen 0,5 und 0.001 %. Bei der Saatgutbehandlung werden im allgemeinen Wirkstoffmengen von 0,001 bis 50 g je Kilogramm Saatgut, vorzugsweise 0,01 bis 10 g. benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele

Beispiel 1

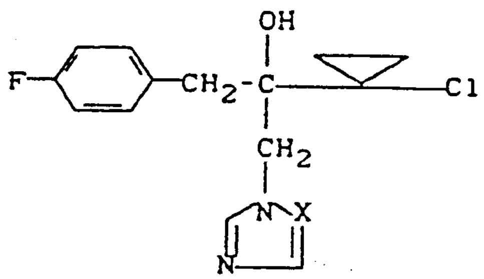

(I-1)

(Verfahren a):

Unter Stickstoffatmosphäre werden 65 g (0,94 Mol) 1,2,4-Triazol und 71 g (0,63 Mol) Kalium-tert.-butylat in 160 ml absolutem Dimethylformamid vorgelegt und auf 80°C erwärmt. Bei dieser Temperatur tropft man unter Rühren eine Lösung von 70,6 g (0,26 Mol) 1-Chlor-2-(1-chlorcyclopropyl)-3-(4-fluorphenyl)-propan-2-ol in 90 ml absolutem Dimethylformamid hinzu. Es wird 6 Stunden bei 100°C nachgerührt und dann durch Abziehen des Verdünnungsmittels unter vermindertem Druck eingeengt. Man nimmt den Rückstand in Essigester auf, wäscht mit Wasser und zieht nach dem Trocknen über Natriumsulfat das Lösungsmittel unter vermindertem Druck ab. Das verbleibende Produkt wird über eine Kieselgel-Säule mit Chloroform als Laufmittel chromatographiert. Man erhält auf diese Weise 28,2 g (39 % der Theorie) an 1-(4-Fluorphenyl)-2-(1-chlor-cyclopropyl)-3-(1,2,4-triazol-1-yl)-propan-2-ol in Form einer Festsubstanz vom Schmelzpunkt 111°C.

Herstellung von Vorprodukten:

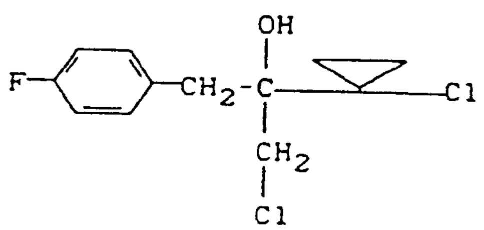

(II-1)

Eine Lösung von 65,7 g (0,35 Mol) 4-Fluorbenzyl-bromid in 430 ml absolutem Diethylether wird bei Raumtemperatur in ein Gemisch aus 9,3 g (0,38 Mol) Magnesium-Spänen in 185 ml Diethylether getropft. Man erhitzt 30 Minuten unter Rückfluß und tropft die entstandene Lösung dann bei -78°C unter Rühren in eine Lösung

von 47,8 g (0,32 Mol) 1-Chlor-1-chloracetyl-cyclopropan in 300 ml absolutem Diethylether ein. Das Reaktionsgemisch wird 4 Stunden bei -78°C gerührt. Danach läßt man langsam auf 0°C erwärmen und tropft dann eine Lösung von 31 ml Essigsäure in 300 ml Diethylether hinzu. Das entstandene Reaktionsgemisch wird auf 1.200 ml Wasser gegossen. Die organische Phase wird abgetrennt, mit wäßriger Natriumhydrogensulfit-Lösung und mit Wasser gewaschen, über Natriumsulfat getrocknet und unter vermindertem Druck eingeengt. Man erhält 70,6 g (86 % der Theorie) an 1-Chlor-2-(1-chlor-cyclopropyl)-3-(4-fluor-phenyl)-propan-2-ol in Form eines öligen Produktes.
$^1$H-NMR (200 MHz, $CDCl_3$): $\delta$ = 0,6-1,2 (m, 4H); 3,05 (d, 1H); 3,15 (d 1H); 3,75 (d, 1H); 4,03 (d, 1H); 7,0 (t, 2H); 7,22-7,37 (m, 2H).

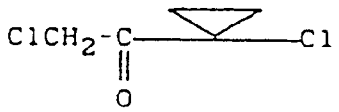

(VIII-1)

Bei Raumtemperatur werden 40,5 ml (0,5 Mol) Sulfurylchlorid unter Rühren langsam in eine Lösung von 54 g (0,46 Mol) 1-Acetyl-1-chlor-cyclopropan in 250 ml Methylenchlorid eingetropft. Es wird zunächst 14 Stunden bei Raumtemperatur und dann 30 Minuten bei 30°C gerührt. Das Reaktionsgemisch wird dann nacheinander mit gesättigter, wäßriger Natriumhydrogencarbonat-Lösung und mit Wasser gewaschen. Danach wird die organische Phase über Natriumsulfat getrocknet und unter vermindertem Druck eingeengt. Man erhält auf diese Weise 51,5 g (74 % der Theorie) an 1-Chlor-1-chloracetyl-cyclopropan in Form einer öligen Substanz.
$^1$H-NMR (60 MHz, $CDCl_3$): $\delta$ = 1,2-1,9 (m, 4H); 4,8 (s, 8H)

Die Verbindung der Formel

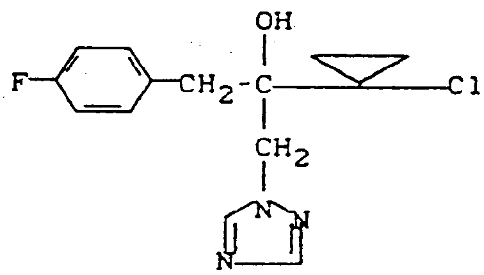

(I-1)

läßt sich auch nach dem erfindungsgemäßen Verfahren (c) herstellen, indem man (1,2,4-Triazol-1-yl-methyl)-(1-chlor-cycloprop-1-yl)-keton mit 4-Fluor-benzyl-magnesium-bromid in Gegenwart von Diethylether umsetzt.

Herstellung des Ausgangsstoffes der Formel:

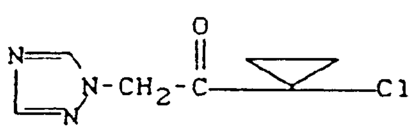

(V-1)

In eine Lösung von 50 g Kaliumcarbonat und 35 g 1,2,4-Triazol in 200 ml Aceton wird bei Raumtemperatur unter Stickstoffatmosphäre und unter Rühren eine Lösung von 60 g 1-Acetyl-1-chlor-cyclopropan in 50 ml Aceton eingetropft. Man erhitzt 8 Stunden unter Rückfluß, engt dann durch Abziehen des Verdünnungsmittels unter vermindertem Druck ein, nimmt dem Rückstand in einem Gemisch aus Essigester und Toluol auf, wäscht mit Wasser, trocknet über Natriumsulfat und engt unter vermindertem Druck ein. Man erhält auf diese Weise 38,9 g an (1,2,4-Triazol-1-yl-methyl)-(1-chlorcycloprop-1-yl)-keton in Form einer Festsubstanz vom Schmelzpunkt 79°C.

$^1$H-NMR (200 MHz, $CDCl_3$):

$\delta$ = 1,50 (m, 2H), 1,78 (m, 2H), 5,62 (s, 2H), 7,98 (s, 1H), 8,14 (s, 1H).

Nach den im Beispiel 1 und in der Beschreibung angegebenen Methoden werden auch die in der folgenden Tabelle aufgeführten Azolylmethyl-cyclopropyl-Derivate der Formel (I) hergestellt.

Tabelle

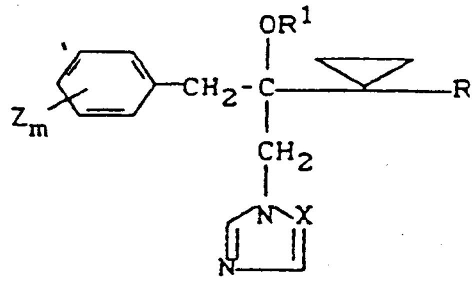

(I)

| Beispiel Nr. | $Z_m$ | $R^1$ | X | R | Schmelzpunkt (°C) |
|---|---|---|---|---|---|
| 2 | 2-F | H | N | Cl | 103 |
| 3 | 2,4-$F_2$ | H | N | Cl | 117 |
| 4 | 2-Cl | H | N | Cl | 108 |
| 5 | 3,4-$Cl_2$ | H | N | Cl | 80-92 |
| 6 | - | H | N | Cl | 81 |
| 7 | 4-Cl | H | N | Cl | 97 |
| 8 | 2,4-$Cl_2$ | H | N | Cl | 159 |
| 9 | 4-$CH_3$ | H | N | Cl | 132 |
| 10 | 3,4-$F_2$ | H | N | Cl | 98 |
| 11 | 4-F | H | CH | Cl | 171 |

In den folgenden Verwendungsbeispielen wurden die Verbindungen der nachstehend angegebenen Formeln als Vergleichssubstanzen eingesetzt:

(A) = 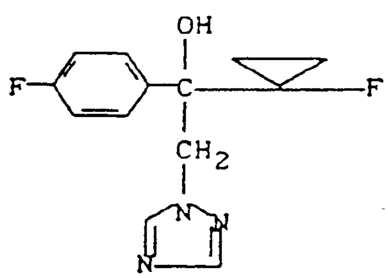

(B) = 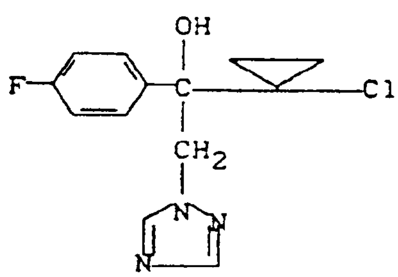

(C) = 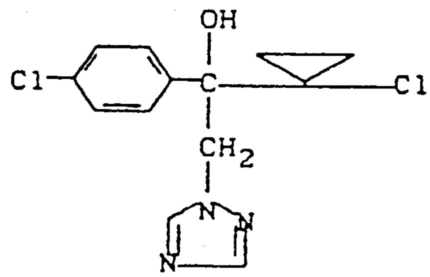

(Bekannt aus EP-A 0 180 136).

(D) = 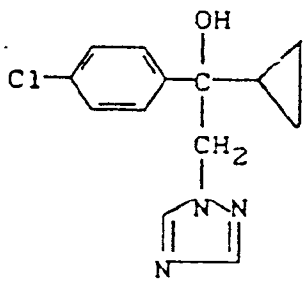

(Bekannt aus EP-A 0 015 756).

Beispiel A

Botrytis-Test (Bohne) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

In diesem Test besitzt die erfindungsgemäße Verbindung (I-1) eine wesentlich bessere Wirksamkeit als die Vergleichssubstanzen (A) und (C).

Beispiel B

Uromyces-Test (Buschbohne) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Uredosporensuspension des Bohnenrosterregers (Uromyces appendiculatus) inokuliert und verbleiben 1 Tag in einer dunklen Feuchtkammer bei 20 bis 22°C und 100 % relativer Luftfeuchtigkeit.

Die Pflanzen werden dann unter intensiver Belichtung für 9 Tage bei 20 bis 22°C und einer relativen Luftfeuchtigkeit von 70 bis 80 % im Gewächshaus aufgestellt.

10 Tage nach der Inokulation erfolgt die Auswertung.

In diesem Test besitzt die erfindungsgemäße Verbindung (I-1) eine wesentlich bessere Wirksamkeit als

die Vergleichssubstanzen (A), (B) und (C).

Beispiel C

Venturia-Test (Apfel) / protektiv

Lösungsmittel; 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

In diesem Test zeigt die erfindungsgemäße Verbindung (I-1) eine wesentlich bessere Wirkung als die Vergleichssubstanzen (A) und (C).

Beispiel D

Pseudocercosporella herpotrichoides-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen an der Halmbasis mit Sporen von Pseudocercosporella herpotrichoides inokuliert.

Die Pflanzen werden in einem Gewächshaus bei einer Temperatur von ca. 10°C und einer relativen Luftfeuchtigkeit von ca. 80 % aufgestellt.

21 Tage nach der Inokulation erfolgt die Auswertung.

In diesem Test zeigt die erfindungsgemäße Verbindung (I-1) eine sehr gute Wirksamkeit.

Beispiel E

Erysiphe-Test (Gerste) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 x 12 Korn 2 cm tief in eine Standarderde. 7 Tage nach der Aussaat, wenn die jungen Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (I-1) eine wesentlich bessere Wirkung als die Vergleichssubstanz (A).

Beispiel F

Leptosphaeria nodorum-Test (Weizen) / protektiv

Lösungsmittel: 100 Gew.-Teile Dimethylformamid
Emulgator: 0,25 Gew.-Teile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht, nach Antrocknen des Spritzbelages werden die Pflanzen mit einer Sporensuspension von Leptosphaeria nodorum besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 15°C und einer relativen Luftfeuchtigkeit von ca. 80 % aufgestellt.

10 Tage nach Inokulation erfolgt die Auswertung.

In diesem Test zeigt die erfindungsgemäße Verbindung (I-1) eine wesentlich bessere Wirksamkeit als die Vergleichssubstanz (D).

Beispiel G

Pyrenophora teres-Test (Gerste) / protektiv

Lösungsmittel: 100 Gew.-Teile Dimethylformamid
Emulgator: 0,25 Gew.-Teile Alkyarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt.

7 Tage nach der Inokulation erfolgt die Auswertung.

In diesem Test zeigt die erfindungsgemäße Verbindung (I-1) eine wesentlich bessere Wirkung als die Vergleichssubstanz (D).

## Patentansprüche

**Patentansprüche für folgende Vertragsstaaten : AT, BE, CH, DE, FR, GB, GR, IT, LI, NL, SE**

**1.** Azolylmethyl-cyclopropyl-Derivate der Formel

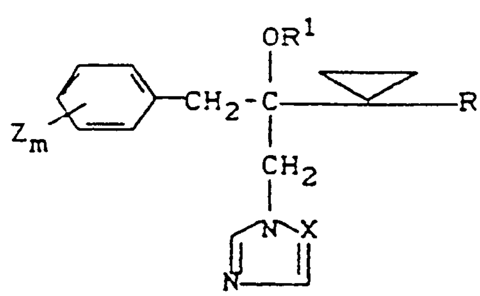

(I)

in welcher

R für Fluor, Chlor, Brom, Alkyl mit 1 bis 6 Kohlenstoffatomen. für gegebenenfalls durch Halogen,

Halogenalkyl mit 1 oder 2 Kohlenstoffatomen und 1 bis 5 Halogenatomen, Alkyl mit 1 bis 4 Kohlensoffatomen und/oder Alkoxy mit 1 bis 4 Kohlenstoffatomen substituiertes Phenyl oder für die Gruppierung -Y-$R^2$ steht, worin

Y für Sauerstoff, Schwefel, SO oder $SO_2$ steht und

$R^2$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,

$R^1$ für Wasserstoff, Alkyl mit 1 bis 6 Kohlenstoffatomen oder Alkylcarbonyl mit 1 bis 6 Kohlenstoffatomen in der Alkylgruppe steht,

X für Stickstoff oder eine CH-Gruppe steht,

Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht und

m für die Zahlen 0, 1, 2 oder 3 steht,

sowie deren Säureadditions-Salze und Metallsalz-Komplexe.

**2.** Verfahren zur Herstellung von Azolylmethylcyclopropyl-Derivaten der Formel

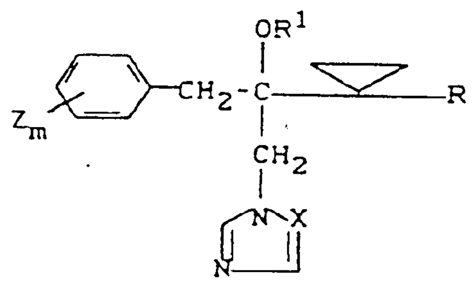

(I)

in welcher
R, $R^1$, X, Z und m die im Anspruch 1 angegebenen Bedeutungen haben,
sowie von deren Säureadditions-Salzen und Metallsalz-Komplexen, dadurch gekennzeichnet, daß man

a) Propanol-Derivate der Formel

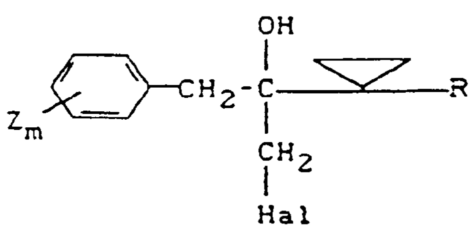

(II)

in welcher
R, Z und m die oben angegebene Bedeutung haben und
Hal für Chlor, Brom oder Iod steht,
mit Azolen der Formel

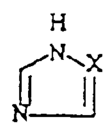

(III)

in welcher
X die oben angegebene Bedeutung hat,
in Gegenwart eines Säurebindemittels und in Gegenwart eines Verdünnungsmittels umsetzt,
oder

b) Oxirane der Formel

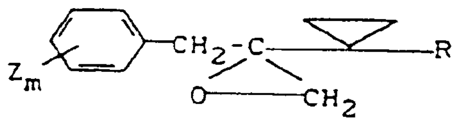

(IV)

in welcher
R, Z und m die oben angegebene Bedeutung haben,
mit Azolen der Formel

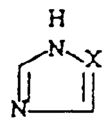

(III)

in welcher
X die oben angegebene Bedeutung hat,
in Gegenwart eines Säurebindemittels und in Gegenwart eines Verdünnungsmittels umsetzt,
oder
c) Azolylmethyl-ketone der Formel

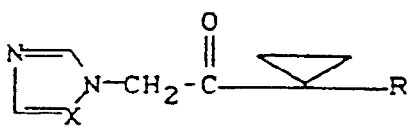

(V)

in welcher
R und X die oben angegebene Bedeutung haben,
mit metallorganischen Verbindungen der Formel

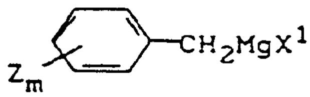

(VI)

in welcher
Z und m die oben angegebene Bedeutung haben und
$X^1$ für Chlor, Brom oder Iod steht,
in Gegenwart eines Verdünnungsmittels umsetzt,
oder
d) Azolylmethyl-cyclopropyl-carbinol-Derivate der Formel

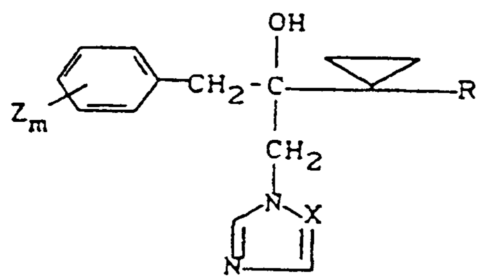

(Ia)

in welcher
R, X, Z und m die oben angegebene Bedeutung haben,

mit starken Basen in Gegenwart eines Verdünnungsmittels umsetzt und die dabei entstehenden Alkoholate der Formel

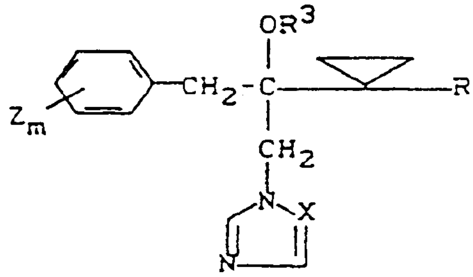

(Ib)

in welcher

P, X, Z und m die oben angegebene Bedeutung haben und

$R^3$ für ein Alkalimetallkation oder für ein quarternäres Ammonium- oder Phosphoniumkation steht.

mit Halogenverbindungen der Formel

$R^4$-Hal' (VII)

in welcher

$R^4$ für Alkyl mit 1 bis 6 Kohlenstoffatomen oder Alkylcarbonyl mit 1 bis 6 Kohlenstoffatomen im Alkylteil steht und

Hal' für Chlor oder Brom steht,

in Gegenwart eines Verdünnungsmittels umsetzt,

und gegebenenfalls anschließend an die so erhaltenen Verbindungen der Formel (I) eine Säure oder ein Metallsalz addiert.

**3.** Fungizide und pflanzenwuchsregulierende Mittel, gekennzeichnet durch einen Gehalt an mindestens einem Azolylmethyl-cyclopropyl-Derivat der Formel (I) gemäß Anspruch 1 bzw. an einem Säureadditions-Salz oder Metallsalz-Komplex eines Azolylmethyl-cyclopropyl-Derivates der Formel (I).

**4.** Verwendung von Azolylmethyl-cyclopropyl-Derivaten der Formel (I) gemäß Anspruch 1 bzw. von deren Säureadditions-Salzen und Metallsalz-Komplexen zur Bekämpfung von Pilzen sowie zur Regulierung des Pflanzenwachstums.

**5.** Propanol-Derivate der Formel

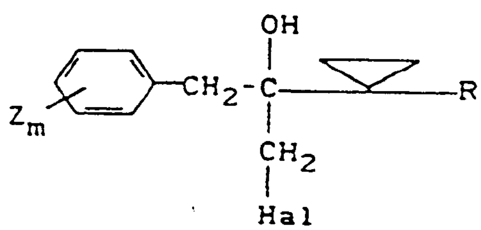

(II)

in welcher

R, Z, Hal und m die oben angegebenen Bedeutungen haben.

**6.** Verfahren zur Herstellung von Propanol-Derivaten der Formel

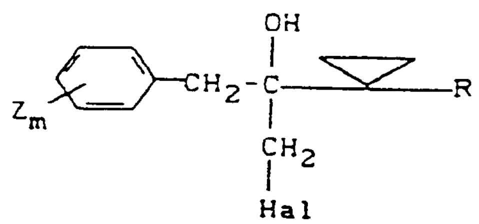

(II)

in welcher
R, Z, Hal und m die oben angegebenen Bedeutungen haben,
dadurch gekennzeichnet, daß man Cyclopropylketone der Formel

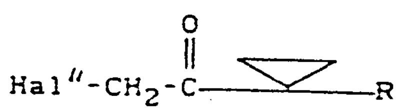

(VIII)

in welcher
R die oben angegebene Bedeutung hat und
Hal" für Chlor oder Brom steht,
mit metallorganischen Verbindungen der Formel

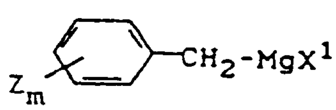

(VI)

in welcher
Z und m die oben angegebene Bedeutung haben und
$X^1$ für Chlor, Brom oder lod steht,
in Gegenwart eines Verdünnungsmittels umsetzt.

**7.** Oxirane der Formel

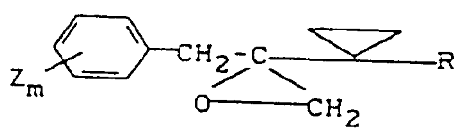

(IV)

in welcher
R, Z, und m die oben angegebenen Bedeutungen haben.

**8.** Verfahren zur Herstellung von Oxiranen der Formel

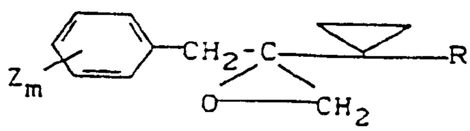

(IV)

in welcher
R, Z und m die oben angegebenen Bedeutungen haben,
dadurch gekennzeichnet, daß man Propanol-Derivate der Formel

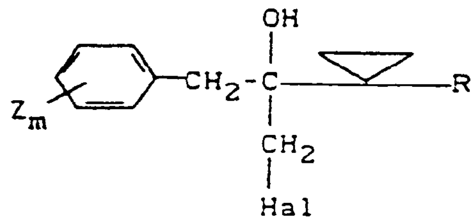

(II)

in welcher
R, Z und m die oben angegebene Bedeutung haben und
Hal für Chlor, Brom oder Iod steht,
mit Basen in Gegenwart eines Verdünnungsmittels umsetzt.

**9.** Azolylmethyl-ketone der Formel

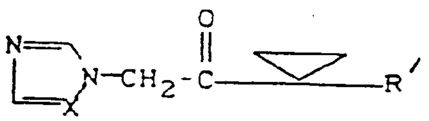

(V-a)

in welcher

R' für Fluor, Chor, Brom, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung -Y-$R^{2'}$ steht, worin

Y für Sauerstoff, Schwefel, SO oder $SO_2$ steht und

$R^{2'}$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht,

und

X für Stickstoff oder eine CH-Gruppe steht.

**10.** Verfahren zur Herstellung von Azolylmethyl-ketonen der Formel

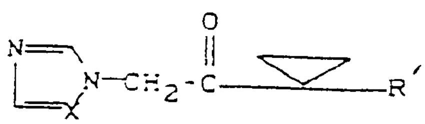

(V-a)

in welcher
R' und X die im Anspruch 9 angegebene Bedeutung haben,
dadurch gekennzeichnet, daß man Cyclopropylketone der Formel

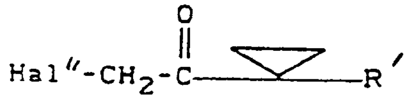

(VIII-a)

in welcher
R' die oben angegebene Bedeutung hat und
Hal" für Chlor oder Brom steht,
mit Azolen der Formel

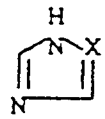

(III)

in welcher
X die oben angegebene Bedeutung hat,
in Gegenwart eines Säurebindemittels und gegebenenfalls in Gegenwart eines Verdünnungsmittels umsetzt.

**Patentansprüche für folgenden Vertragsstaat : ES**

1. Verfahren zur Herstellung von Azolylmethylcyclopropyl-Derivaten der Formel

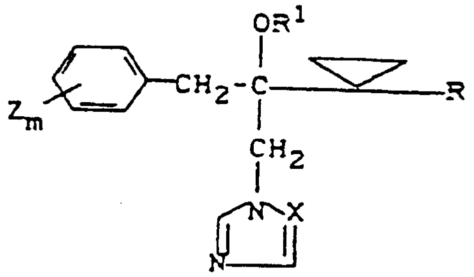

(I)

in welcher

R für Fluor, Chlor, Brom, Alkyl mit 1 bis 6 Kohlenstoffatomen, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung -Y-$R^2$ steht, worin
Y für Sauerstoff, Schwefel, SO oder $SO_2$ steht und
$R^2$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,

$R^1$ für Wasserstoff, Alkyl mit 1 bis 6 Kohlenstoffatomen oder Alkylcarbonyl mit 1 bis 6 Kohlenstoffatomen in der Alkylgruppe steht.

X für Stickstoff oder eine CH-Gruppe steht

Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy steht und

m für die Zahlen 0, 1, 2 oder 3 steht,

sowie von deren Säureadditions-Salzen und Metallsalz-Komplexen, dadurch gekennzeichnet, daß man

a) Propanol-Derivate der Formel

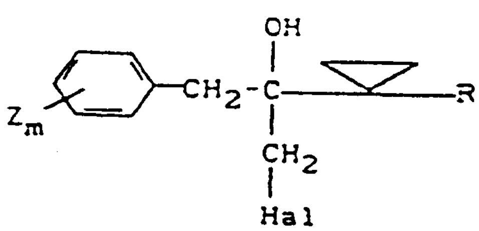

(II)

in welcher
R, Z und m die oben angegebene Bedeutung haben und
Hal für Chlor, Brom oder Iod steht,
mit Azolen der Formel

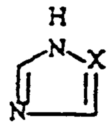 (III)

in welcher
X die oben angegebene Bedeutung hat,
in Gegenwart eines Säurebindemittels und in Gegenwart eines Verdünnungsmittels umsetzt,
oder
b) Oxirane der Formel

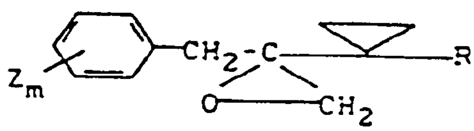 (IV)

in welcher
R, Z und m die oben angegebene Bedeutung haben,
mit Azolen der Formel

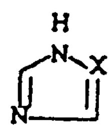 (III)

in welcher
X die oben angegebene Bedeutung hat,
in Gegenwart eines Säurebindemittels und in Gegenwart eines Verdünnungsmittels umsetzt,
oder
c) Azolylmethyl-ketone der Formel

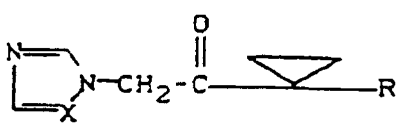 (V)

in welcher
R und X die oben angegebene Bedeutung haben,
mit metallorganischen Verbindungen der Formel

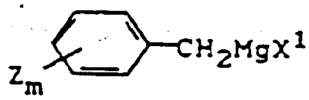 (VI)

in welcher
Z und m die oben angegebene Bedeutung haben und
$X^1$ für Chlor, Brom oder Iod steht,
in Gegenwart eines Verdünnungsmittels umsetzt,
oder
d) Azolylmethyl-cyclopropyl-carbinol-Derivate der Formel

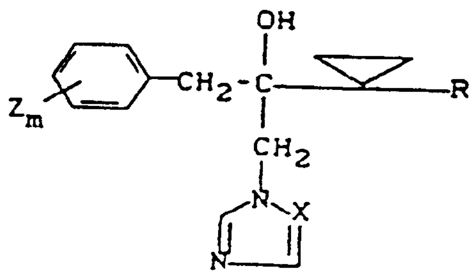

(Ia)

in welcher
R, X, Z und m die oben angegebene Bedeutung haben,
mit starken Basen in Gegenwart eines Verdünnungsmittels umsetzt und die dabei entstehenden Alkoholate der Formel

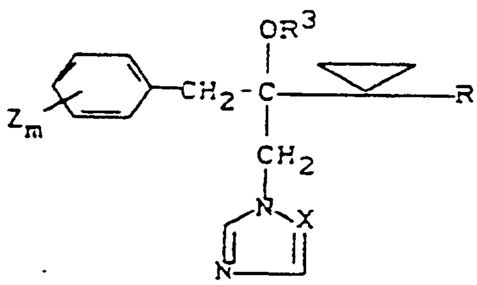

(Ib)

in welcher
R, X, Z und m die oben angegebene Bedeutung haben und
$R^3$ für ein Alkalimetallkation oder für ein quarternäres Ammonium- oder Phosphoniumkation steht,
mit Halogenverbindungen der Formel

$R^4$-Hal' (VII)

in welcher
$R^4$ für Alkyl mit 1 bis 6 Kohlenstoffatomen oder Alkylcarbonyl mit 1 bis 6 Kohlenstoffatomen im Alkylteil steht und
Hal' für Chlor oder Brom steht,
in Gegenwart eines Verdünnungsmittels umsetzt,
und gegebenenfalls anschließend an die so erhaltenen Verbindungen der Formel (I) eine Säure oder ein Metallsalz addiert.

2. Fungizide und pflanzenwuchsregulierende Mittel, gekennzeichnet durch einen Gehalt an mindestens einem Azolylmethyl-cyclopropyl-Derivat der Formel (I) gemäß Anspruch 1 bzw. an einem Säureadditions-Salz oder Metallsalz-Komplex eines Azolylmethyl-cyclopropyl-Derivates der Formel (I).

3. Verwendung von Azolylmethyl-cyclopropyl-Derivaten der Formel (I) gemäß Anspruch 1 bzw. von deren Säureadditions-Salzen und Metallsalz-Komplexen zur Bekämpfung von Pilzen sowie zur Regulierung des Pflanzenwachstums.

4. Verfahren zur Herstellung von Propanol-Derivaten der Formel

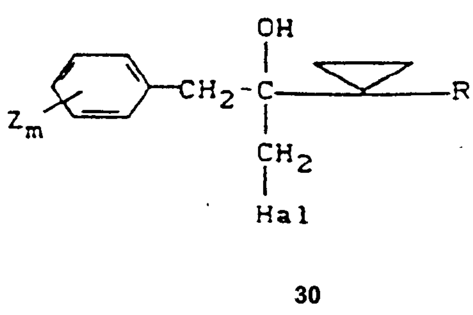

(II)

in welcher
R, Z, Hal und m die oben angegebenen Bedeutungen haben,
dadurch gekennzeichnet, daß man Cyclopropylketone der Formel

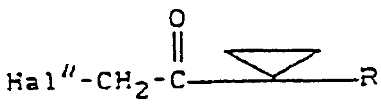

(VIII)

in welcher
R die oben angegebene Bedeutung hat und
Hal" für Chlor oder Brom steht,
mit metallorganischen Verbindungen der Formel

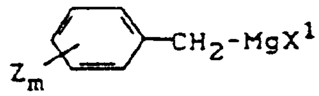

(VI)

in welcher
Z und m die oben angegebene Bedeutung haben und
$X^1$ für Chlor, Brom oder Iod steht,
in Gegenwart eines Verdünnungsmittels umsetzt.

5. Verfahren zur Herstellung von Oxiranen der Formel

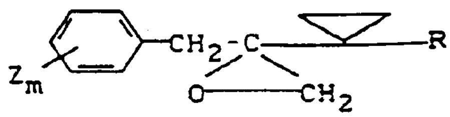

(IV)

in welcher
R, Z und m die oben angegebenen Bedeutungen haben,
dadurch gekennzeichnet, daß man Propanol-Derivate der Formel

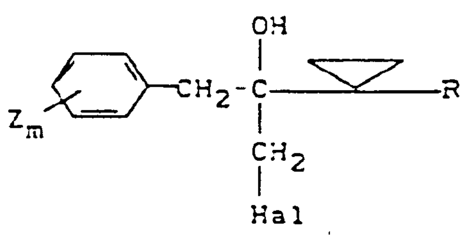

(II)

in welcher
R, Z und m die oben angegebene Bedeutung haben und
Hal für Chlor, Brom oder Iod steht,
mit Basen in Gegenwart eines Verdünnungsmittels umsetzt.

6. Verfahren zur Herstellung von Azolylmethyl-ketonen der Formel

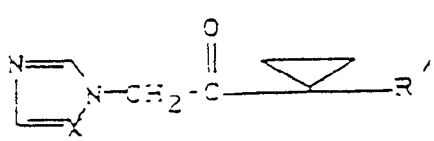

(V-a)

in welcher

R' für Fluor, Chor, Brom, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung -Y-$R^{2'}$ steht, worin

Y für Sauerstoff, Schwefel, SO oder $SO_2$ steht und

$R^{2'}$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht,

und

X für Stickstoff oder eine OH-Gruppe steht,

dadurch gekennzeichnet, daß man Cyclopropylketone der Formel

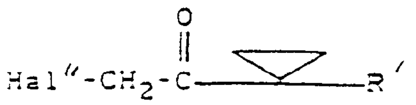

(VIII-a)

in welcher
R' die oben angegebene Bedeutung hat und
Hal" für Chlor oder Brom steht,
mit Azolen der Formel

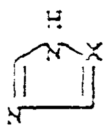

(III)

in welcher
X die oben angegebene Bedeutung hat,
in Gegenwart eines Säurebindemittels und gegebenenfalls in Gegenwart eines Verdünnungsmittels umsetzt.

## Claims

### Claims for the following Contracting States : AT, BE, CH, DE, FR, GB, GR, IT, LI, NL, SE

**1.** Azolylmethyl-cyclopropyl derivatives of the formula

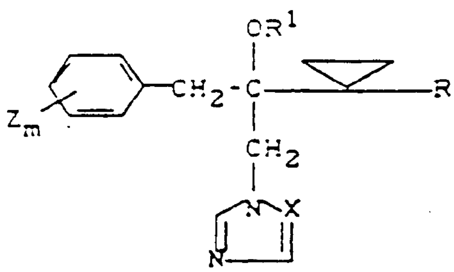

**(I)**

in which

R represents fluorine, chlorine, bromine, alkyl having 1 to 6 carbon atoms, or represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or represents the grouping -Y-$R^2$, wherein

Y represents oxygen, sulphur, SO or $SO_2$ and

$R^2$ represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms,

$R^1$ represents hydrogen, alkyl having 1 to 6 carbon atoms or alkylcarbonyl having 1 to 6 carbon atoms

in the alkyl group,

X represents nitrogen or a CH group,

Z represents fluorine, chlorine, bromine, methyl, ethyl, isopropyl, tert-butyl, methoxy, methylthio, trifluoromethyl, trifluoromethoxy, trifluoromethylthio, phenyl which is optionally substituted by fluorine, chlorine and/or methyl, or represents phenoxy which is optionally substituted by fluorine, chlorine and/or methyl and

m represents the number 0, 1, 2 or 3,

and acid addition salts and metal salt complexes thereof.

**2.** Process for the preparation of azolylmethylcyclopropyl derivatives of the formula

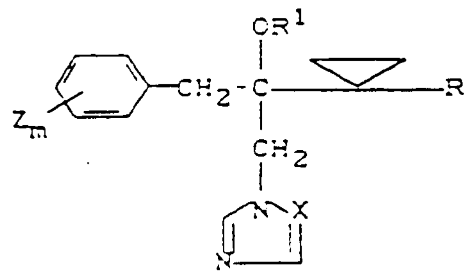

(I)

in which

R, $R^1$, X, Z and m have the meanings given in Claim 1,

and acid addition salts and metal salt complexes thereof, characterised in that

a) propanol derivatives of the formula

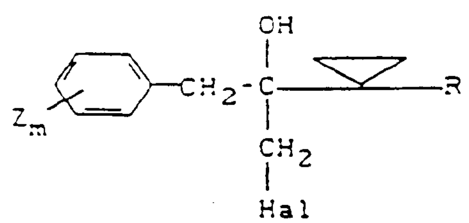

(II)

in which

R, Z and m have the abovementioned meaning and

Hal represents chlorine, bromine or iodine,

are reacted with azoles of the formula

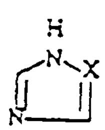

(III)

in which

X has the abovementioned meaning,

in the presence of an acid-binding agent and in the presence of a diluent,

or

b) oxiranes of the formula

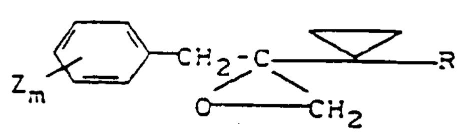

(IV)

in which
R, Z and m have the abovementioned meaning,
are reacted with azoles of the formula

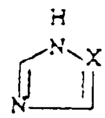

(III)

in which
X has the abovementioned meaning,
in the presence of an acid-binding agent and in the presence of a diluent,
or
c) azolylmethyl ketones of the formula

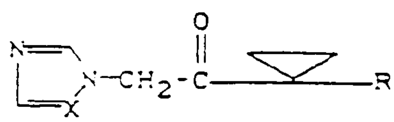

(V)

in which
R and X have the abovementioned meaning,
are reacted with organometallic compounds of the formula

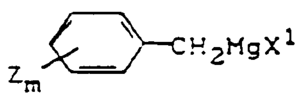

(VI)

in which
Z and m have the abovementioned meaning and
$X^1$ represents chlorine, bromine or iodine, in the presence of a diluent
or
d) azolylmethyl-cyclopropyl-carbinol derivatives of the formula

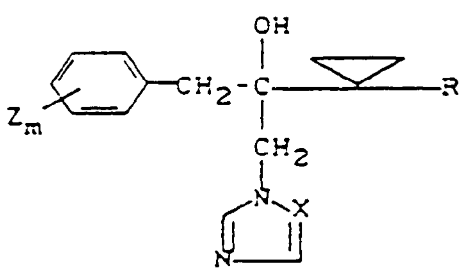

(Ia)

in which
R, X, Z and m have the abovementioned meaning,
are reacted with strong bases in the presence of a diluent, and the alcoholates thereby formed, of the formula

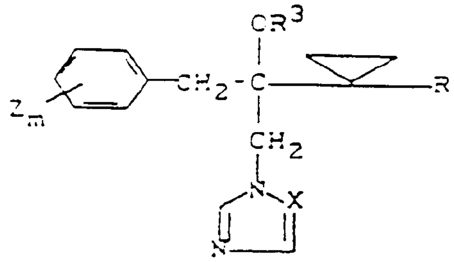

(Ib)

in which

R, X, Z and m have the abovementioned meaning and

$R^3$ represents an alkali metal cation or a quaternary ammonium or phosphonium cation,

are reacted with halogen compounds of the formula

$R^4$-Hal' (VII)

in which

$R^4$ represents alkyl having 1 to 6 carbon atoms or alkylcarbonyl having 1 to 6 carbon atoms in the alkyl moiety and

Hal' represents chlorine or bromine,

in the presence of a diluent,

and, if appropriate, an acid or a metal salt is then added onto the compounds of the formula (I) thus obtained.

3. Fungicidal and plant growth-regulating agents, characterised in that they contain at least one azolylmethyl-cyclopropyl derivative of the formula (I) according to Claim 1 or an acid addition salt or metal salt complex of an azolylmethyl-cyclopropyl derivative of the formula (I).

4. Use of azolylmethyl-cyclopropyl derivatives of the formula (I) according to Claim 1 or of acid addition salts and metal salt complexes thereof for combating fungi and for regulating plant growth.

5. Propanol derivatives of the formula

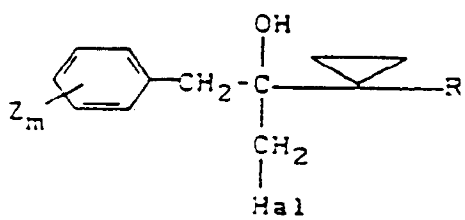

(II)

in which

R, Z, Hal and m have the abovementioned meanings.

6. Process for the preparation of propanol derivatives of the formula

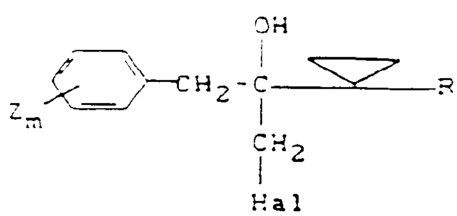

(II)

in which

R, Z, Hal and m have the abovementioned meanings,

characterised in that cyclopropyl ketones of the formula

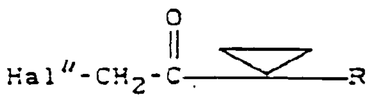

(VIII)

in which
R has the abovementioned meaning and
Hal" represents chlorine or bromine
are reacted with organometallic compounds of the formula

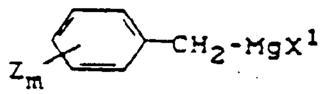

(VI)

in which
Z and m have the abovementioned meaning and
$X^1$ represents chlorine, bromine or iodine,
in the presence of a diluent.

7. Oxiranes of the formula

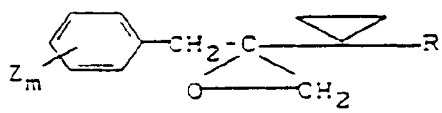

(IV)

in which
R, Z and m have the abovementioned meanings.

8. Process for the preparation of oxiranes of the formula

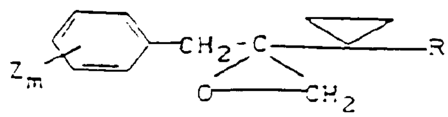

(IV)

in which
R, Z and m have the abovementioned meanings,
characterised in that propanol derivatives of the formula

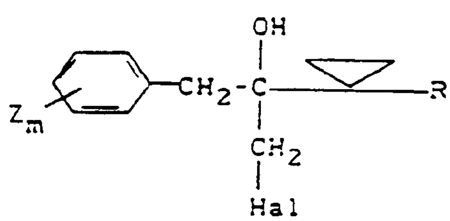

(II)

in which
R, Z and m have the abovementioned meaning and
Hal represents chlorine, bromine or iodine,
are reacted with bases in the presence of a diluent.

9. Azolylmethyl ketones of the formula

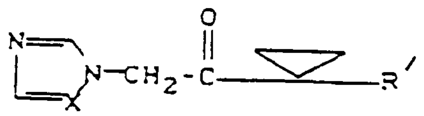

(V-a)

in which

$R^1$ represents fluorine, chlorine, bromine, or phenyl which is optionally substituted by one to three identical or different substituents from the group comprising fluorine, chlorine, bromine, trifluoromethyl, methyl, ethyl, methoxy and/or ethoxy, or represents the grouping $-Y-R^{2'}$,
wherein

Y represents oxygen, sulphur, SO or $SO_2$ and

$R^{2'}$ represents phenyl which is optionally substituted by one to three identical or different substituents from the group comprising fluorine, chlorine, bromine, trifluoromethyl, methyl, ethyl, methoxy and/or ethoxy,

and

X represents nitrogen or a CH group.

**10.** Process for the preparation of azolylmethyl ketones of the formula

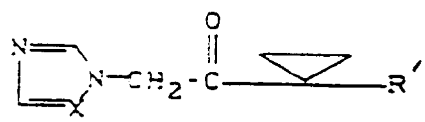

(V-a)

in which
R' and X have the meaning given in Claim 9,
characterised in that cyclopropyl ketones of the formula

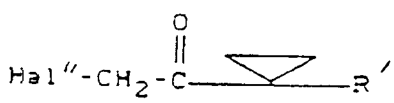

(VIII-a)

in which
R' has the abovementioned meaning and
Hal" represents chlorine or bromine,
are reacted with azoles of the formula

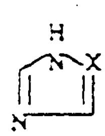

(III)

in which
X has the abovementioned meaning,
in the presence of an acid-binding agent and, if appropriate, in the presence of a diluent.

**Claims for the following Contracting State : ES**

**1.** Process for the preparation of azolylmethylcyclopropyl derivatives of the formula

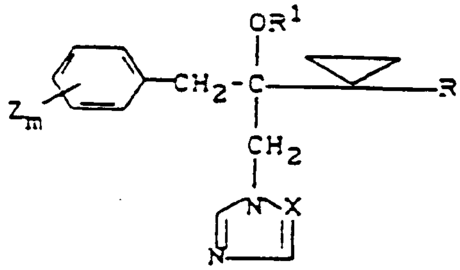

(I)

in which

R represents fluorine, chlorine, bromine, alkyl having 1 to 6 carbon atoms, or represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or represents the grouping -Y-$R^2$, wherein

Y represents oxygen, sulphur, SO or $SO_2$ and

$R^2$ represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms,

$R^1$ represents hydrogen, alkyl having 1 to 6 carbon atoms or alkylcarbonyl having 1 to 6 carbon atoms in the alkyl group,

X represents nitrogen or a CH group,

Z represents fluorine, chlorine, bromine, methyl, ethyl, isopropyl, tert-butyl, methoxy, methylthio, trifluoromethyl, trifluoromethoxy, trifluoromethylthio, phenyl which is optionally substituted by fluorine, chlorine and/or methyl, or represents phenoxy which is optionally substituted by fluorine, chlorine and/or methyl and

m represents the number 0, 1, 2 or 3,

and acid addition salts and metal salt complexes thereof, characterised in that

a) propanol derivatives of the formula

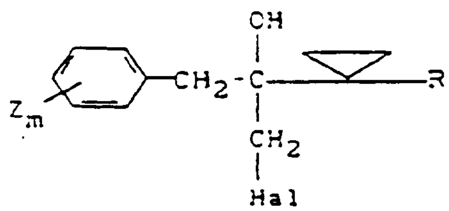

(II)

in which
R, Z and m have the abovementioned meaning and
Hal represents chlorine, bromine or iodine,
are reacted with azoles of the formula

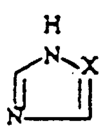

(III)

in which
X has the abovementioned meaning,
in the presence of an acid-binding agent and in the presence of a diluent,
or

b) oxiranes of the formula

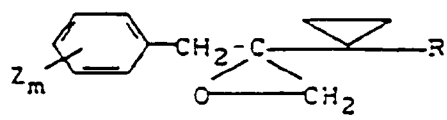

(IV)

in which
R, Z and m have the abovementioned meaning,
are reacted with azoles of the formula

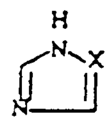

(III)

in which
X has the abovementioned meaning,
in the presence of an acid-binding agent and in the presence of a diluent,
or
c) azolylmethyl ketones of the formula

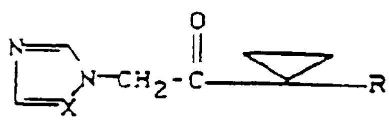

(V)

in which
R and X have the abovementioned meaning,
are reacted with organometallic compounds of the formula

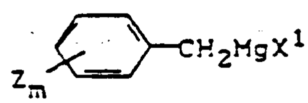

(VI)

in which
Z and m have the abovementioned meaning and
$X^1$ represents chlorine, bromine or iodine,
in the presence of a diluent
or
d) azolylmethyl-cyclopropyl-carbinol derivatives of the formula

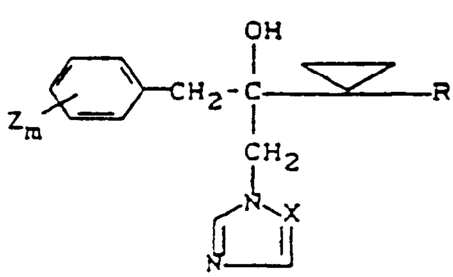

(Ia)

in which
R, X, Z and m have the abovementioned meaning,
are reacted with strong bases in the presence of a diluent, and the alcoholates thereby formed, of the formula

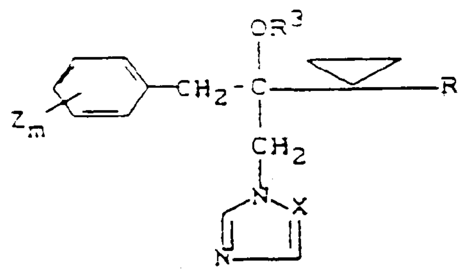

(Ib)

in which
R, X, Z and m have the abovementioned meaning and
$R^3$ represents an alkali metal cation or a quaternary ammonium or phosphonium cation,
are reacted with halogen compounds of the formula

$R^4$-Hal' (VII)

in which
$R^4$ represents alkyl having 1 to 6 carbon atoms or alkylcarbonyl having 1 to 6 carbon atoms in the alkyl moiety and
Hal' represents chlorine or bromine,
in the presence of a diluent,
and, if appropriate, an acid or a metal salt is then added onto the compounds of the formula (I) thus obtained.

2. Fungicidal and plant growth-regulating agents, characterised in that they contain at least one azolylmethyl-cyclopropyl derivative of the formula (I) according to Claim 1 or an acid addition salt or metal salt complex of an azolylmethyl-cyclopropyl derivative of the formula (I).

3. Use of azolylmethyl-cyclopropyl derivatives of the formula (I) according to Claim 1 or of acid addition salts and metal salt complexes thereof for combating fungi and for regulating plant growth.

4. Process for the preparation of propanol derivatives of the formula

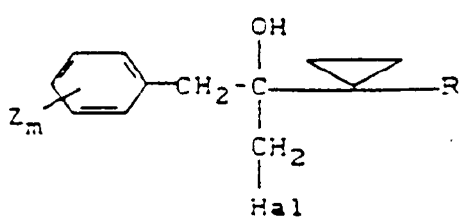

(II)

in which
R, Z, Hal and m have the abovementioned meanings,
characterised in that cyclopropyl ketones of the formula

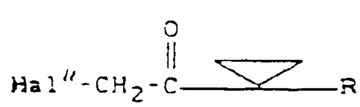

(VIII)

in which
R has the abovementioned meaning and
Hal" represents chlorine or bromine
are reacted with organometallic compounds of the formula

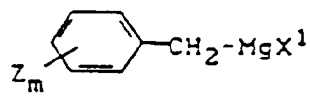

(VI)

in which
Z and m have the abovementioned meaning and
$X^1$ represents chlorine, bromine or iodine,
in the presence of a diluent.

**5.** Process for the preparation of oxiranes of the formula

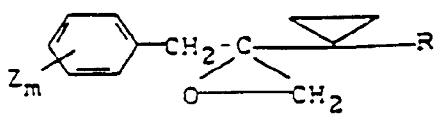

(IV)

in which
R, Z and m have the abovementioned meanings,
characterised in that propanol derivatives of the formula

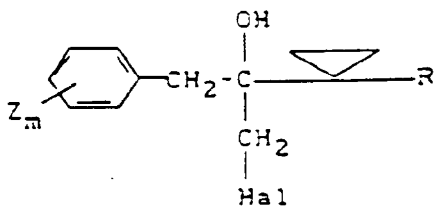

(II)

in which
R, Z and m have the abovementioned meanings and Hal represents chlorine, bromine or iodine,
are reacted with bases in the presence of a diluent.

**6.** Process for the preparation of azolylmethyl ketones of the formula

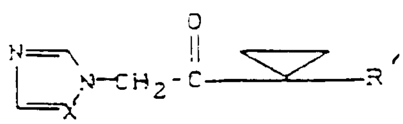

(V-a)

in which

R' represents fluorine, chlorine, bromine, or phenyl which is optionally substituted by one to three identical or different substituents from the group comprising fluorine, chlorine, bromine, trifluoromethyl, methyl, ethyl, methoxy, and/or ethoxy, or represents the grouping -Y-$R^{2'}$, wherein

Y represents oxygen, sulphur, SO or $SO_2$ and

$R^{2'}$ represents phenyl which is optionally substituted by one to three identical or different substituents from the group comprising fluorine, chlorine, bromine, trifluoromethyl, methyl, ethyl, methoxy and/or ethoxy,

and

X represents nitrogen or a CH group,

characterised in that cyclopropyl ketones of the formula

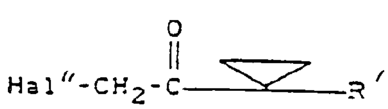

(VIII-a)

in which
R' has the abovementioned meaning and
Hal" represents chlorine or bromine,
are reacted with azoles of the formula

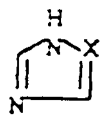

(III)

in which
X has the abovementioned meaning,
in the presence of an acid-binding agent and, if appropriate, in the presence of a diluent.

**Revendications**

**Revendications pour les Etats contractants suivants : AT, BE, CH, DE, FR, GB, GR, IT, LI, NL, SE**

1. Dérivés d'azolylméthyl-cyclopropyle de formule

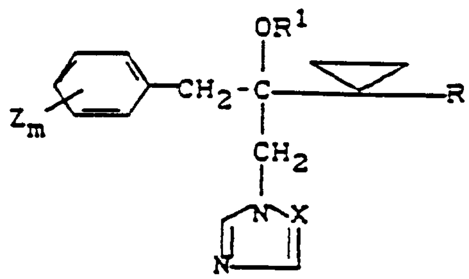

(I)

dans laquelle

R représente un atome de fluor, un atome de chlore, un atome de brome, un groupe alkyle contenant de 1 à 6 atomes de carbone, un groupe phényle éventuellement substitué par un atome d'halogène, par un groupe halogénalkyle contenant 1 ou 2 atomes de carbone et de 1 à 5 atomes d'halogène, par un groupe alkyle contenant de 1 à 4 atomes de carbone et/ou par un groupe alcoxy contenant de 1 à 4 atomes de carbone ou encore le groupement -Y-$R^2$, où

Y représente un atome d'oxygène, un atome de soufre, SO ou $SO_2$ et

$R^2$ représente un groupe phényle éventuellement substitué par un atome d'halogène, par un groupe halogénalkyle contenant 1 ou 2 atomes de carbone et de 1 à 5 atomes d'halogène, par un groupe alkyle contenant de 1 à 4 atomes de carbone et/ou par un groupe alcoxy contenant de 1 à 4 atomes de carbone,

$R^1$ représente un atome d'hydrogène, un groupe alkyle contenant de 1 à 6 atomes de carbone ou un groupe alkylcarbonyle contenant de 1 à 6 atomes de carbone dans le groupe alkyle,

X représente un atome d'azote ou un groupe CH,

Z représente un atome de fluor, un atome de chlore, un atome de brome, un groupe méthyle, un groupe éthyle, un groupe isopropyle, un groupe tert.-butyle, un groupe méthoxy, un groupe méthylthio, un groupe trifluorométhyle, un groupe trifluorométhoxy, un groupe trifluorométhylthio, un groupe phényle éventuellement substitué par un atome de fluor, par un atome de chlore et/ou par un groupe méthyle ou encore un groupe phénoxy éventuellement substitué par un atome de fluor, par un atome de chlore et/ou par un groupe méthyle et

m représente les chiffres 0, 1, 2 ou 3,

ainsi que leurs sels d'addition d'acides et leurs complexes de sels métalliques.

2. Procédé pour la préparation de dérivés d'azolylméthyl-cyclopropyle de formule

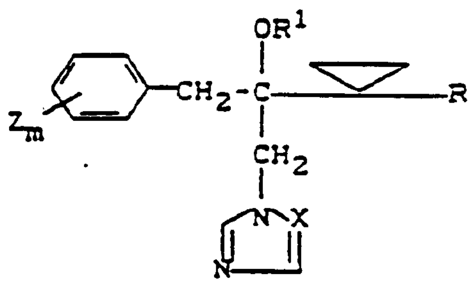

(I)

dans laquelle

R, $R^1$, X, Z et m ont les significations mentionnées à la revendication 1,

ainsi que de leurs sels d'addition d'acides et de leurs complexes de sels métalliques, **caractérisé en ce qu'**on fait réagir

a) des dérivés de propanol de formule

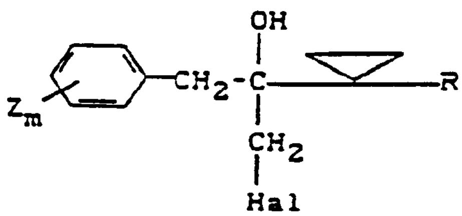

(II)

dans laquelle

R, Z et m ont la signification mentionnée ci-dessus et

Hal représente un atome de chlore, un atome de brome ou un atome d'iode, avec des azoles de formule

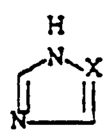

(III)

dans laquelle

X a la signification mentionnée ci-dessus,

en présence d'un agent fixateur d'acides et en présence d'un diluant

ou

b) des oxirannes de formule

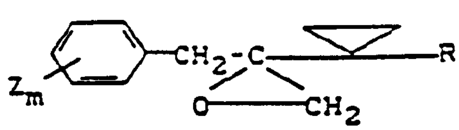

(IV)

dans laquelle

R, Z et m ont la signification mentionnée ci-dessus,

avec des azoles de formule

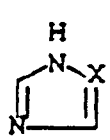

(III)

dans laquelle

X a la signification mentionnée ci-dessus,

en présence d'un agent fixateur d'acides et en présence d'un diluant

ou

c) des azolylméthyl-cétones de formule

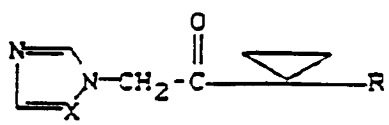

(V)

dans laquelle

R et X ont la signification mentionnée ci-dessus,

avec des composés organométalliques de formule

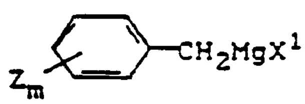

(VI)

dans laquelle

Z et m ont la signification mentionnée ci-dessus

et

$X^1$ représente un atome de chlore, un atome de brome ou un atome d'iode,

en présence d'un diluant

ou

d) des dérivés d'azolylméthyl-cyclopropylcarbinol de formule

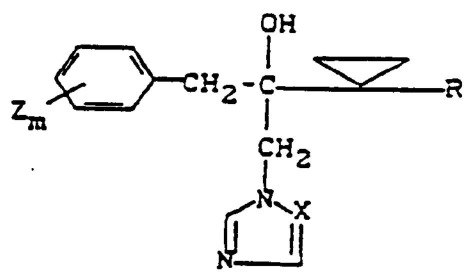

(Ia)

dans laquelle

R, X, Z et m ont la signification mentionnée ci-dessus,

avec des bases fortes en présence d'un diluant, et en faisant réagir les alcoolates que l'on obtient en l'occurrence et répondant à la formule

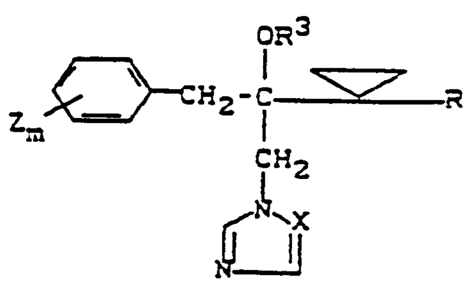

(Ib)

dans laquelle

R, X, Z et m ont la signification mentionnée ci-dessus et

$R^3$ représente un cation de métal alcalin ou un cation de phosphonium ou d'ammonium quaternaire,

avec des composés halogénés de formule

$R^4$-Hal' (VII)

où

$R^4$ représente un groupe alkyle contenant de 1 à 6 atomes de carbone ou un groupe alkylcarbonyle contenant de 1 à 6 atomes de carbone dans la fraction alkyle et

Hal' représente un atome de chlore ou un atome de brome,

en présence d'un diluant

et ensuite, on ajoute éventuellement un acide ou un sel métallique aux composés de formule (I) ainsi obtenus.

3. Fongicides et agents régulateurs de la croissance des plantes, **caractérisés par** une teneur en au moins un dérivé d'azolylméthyl-cyclopropyle de formule (I) selon la revendication 1 ou en un sel d'addition d'acide ou encore d'un complexe de sel métallique d'un dérivé d'azolylméthylcyclopropyle de formule (I).

4. Utilisation de dérivés d'azolylméthylcyclopropyle de formule (I) selon la revendication 1 ou de leurs sels d'addition d'acides ou encore de leurs complexes de sels métalliques, pour lutter contre les champignons, ainsi que pour régulariser la croissance des plantes.

5. Dérivés de propanol de formule

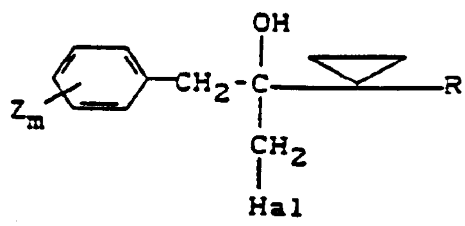

(II)

dans laquelle

R, Z, Hal et m ont les significations mentionnées ci-dessus.

6. Procédé pour la préparation de dérivés de propanol de formule

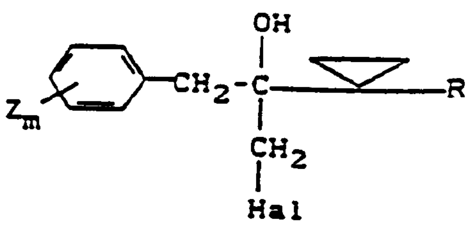

(II)

dans laquelle

R, Z, Hal et m ont les significations mentionnées ci-dessus,

**caractérisé en ce qu'**on fait réagir des cyclopropyl-cétones de formule

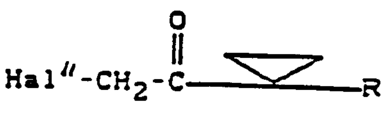

(VIII)

dans laquelle

R a la signification mentionnée ci-dessus et

Hal" représente un atome de chlore ou un atome de brome

avec des composés organométalliques de formule

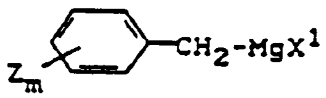

(VI)

dans laquelle

Z et m ont la signification mentionnée ci-dessus et

$X^1$ représente un atome de chlore, un atome de brome ou un atome d'iode,

en présence d'un diluant.

**7.** Oxirannes de formule

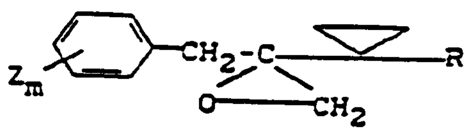

(IV)

dans laquelle

R, Z et m ont les significations mentionnées ci-dessus.

**8.** Procédé pour la préparation d'oxirannes de formule

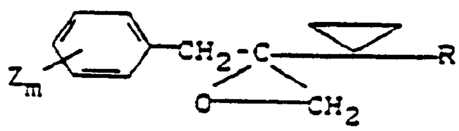

(IV)

dans laquelle

R, Z et m ont les significations mentionnées ci-dessus,

**caractérisé en ce qu'**on fait réagir des dérivés de propanol de formule

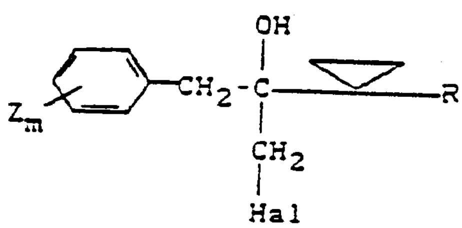

(II)

dans laquelle

R, Z et m ont la signification mentionnée ci-dessus et

Hal représente un atome de chlore, un atome de brome ou un atome d'iode,

avec des bases en présence d'un diluant.

**9.** Azolylméthyl-cétones de formule

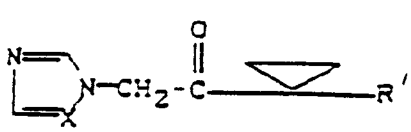

(V-a)

dans laquelle

R' représente un atome de fluor, un atome de chlore, un atome de brome, un groupe phényle éventuellement substitué 1 à 3 fois de manière identique ou différente par un atome de fluor, un atome

de chlore, un atome de brome, un groupe trifluorométhyle, un groupe méthyle, un groupe éthyle, un groupe méthoxy et/ou un groupe éthoxy, ou représente le groupement -Y-$R^{2'}$ où

Y représente un atome d'oxygène, un atome de soufre, SO ou $SO_2$, et

$R^{2'}$ représente un groupe phényle éventuellement substitué 1 à 3 fois de manière identique ou différente par un atome de fluor, un atome de chlore, un atome de brome, un groupe trifluorométhyle, un groupe méthyle, un groupe éthyle, un groupe méthoxy et/ou un groupe éthoxy, et

X représente un atome d'azote ou un groupe CH.

**10.** Procédé pour la préparation d'azolylméthylcétones de formule

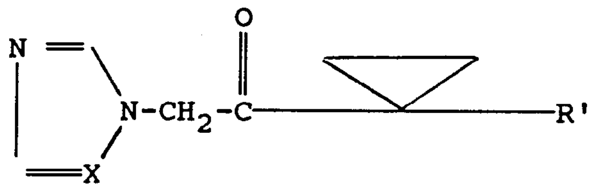

(V-a)

dans laquelle

R' et X ont les significations mentionnées dans la revendication 9,

**caractérisé en ce qu'**on fait réagir des cyclopropylcétones de formule

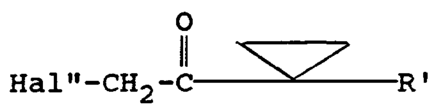

(VIII-a)

dans laquelle

R' a la signification mentionnée ci-dessus et

Hal" représente un atonie de chlore ou un atome de brome

avec des azoles de formule

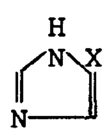

(III)

dans laquelle

X a la signification mentionnée ci-dessus,

en présence d'un agent fixateur d'acides et éventuellement en présence d'un diluant.

**Revendications pour l'Etat contractant suivant : ES**

**1.** Procédé pour la préparation de dérivés d'azolylméthyl-cyclopropyle de formule

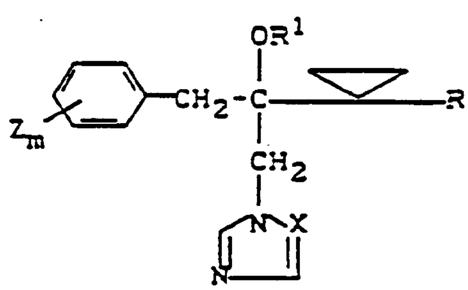

(I)

dans laquelle

R représente un atome de fluor, un atome de chlore, un atome de brome, un groupe alkyle contenant de 1 à 6 atomes de carbone, un groupe phényle éventuellement substitué par un atome d'halogène, par un groupe halogénalkyle contenant 1 ou 2 atomes de carbone et de 1 à 5 atomes d'halogène, par un groupe alkyle contenant de 1 à 4 atomes de carbone et/ou par un groupe alcoxy contenant de 1 à 4 atomes de carbone ou encore le groupement $-Y-R^2$, où

Y représente un atome d'oxygène, un atome de soufre, SO ou $SO_2$ et

$R^2$ représente un groupe phényle éventuellement substitué par un atome d'halogène, par un groupe halogénalkyle contenant 1 ou 2 atomes de carbone et de 1 à 5 atomes d'halogène, par un groupe alkyle contenant de 1 à 4 atomes de carbone et/ou par un groupe alcoxy contenant de 1 à 4 atomes de carbone,

$R^1$ représente un atome d'hydrogène, un groupe alkyle contenant de 1 à 6 atomes de carbone ou un groupe alkylcarbonyle contenant de 1 à 6 atomes de carbone dans le groupe alkyle,

X représente un atome d'azote ou un groupe CH,

Z représente un atome de fluor, un atome de chlore, un atome de brome, un groupe méthyle, un groupe éthyle, un groupe isopropyle, un groupe tert.-butyle, un groupe méthoxy, un groupe méthylthio, un groupe trifluorométhyle, un groupe trifluorométhoxy, un groupe trifluorométhylthio, un groupe phényle éventuellement substitué par un atome de fluor, par un atome de chlore et/ou par un groupe méthyle ou encore un groupe phénoxy éventuellement substitué par un atome de fluor, par un atome de chlore et/ou par un groupe méthyle et

m représente les chiffres 0, 1, 2 ou 3,

ainsi que de leurs sels d'addition d'acides et de leurs complexes de sels métalliques, **caractérisé en ce qu'**on fait réagir

a) des dérivés de propanol de formule

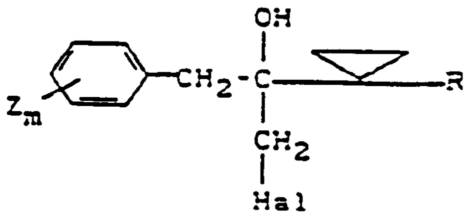

(II)

dans laquelle

R, Z et m ont la signification mentionnée ci-dessus et

Hal représente un atome de chlore, un atome de brome ou un atome d'iode,

avec des azoles de formule

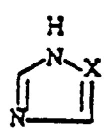

(III)

dans laquelle

X a la signification mentionnée ci-dessus,

en présence d'un agent fixateur d'acides et en présence d'un diluant

ou

b) des oxirannes de formule

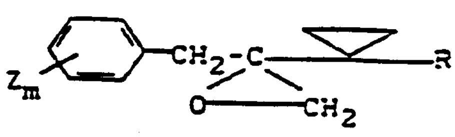

(IV)

dans laquelle

R, Z et m ont la signification mentionnée ci-dessus,

avec des azoles de formule

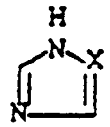

(III)

dans laquelle

X a la signification mentionnée ci-dessus,

en présence d'un agent fixateur d'acides et en présence d'un diluant

ou

c) des azolylméthyl-cétones de formule

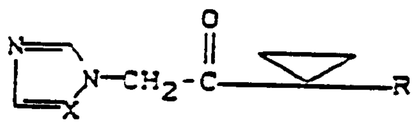

(V)

dans laquelle

R et X ont la signification mentionnée ci-dessus,

avec des composés organométalliques de formule

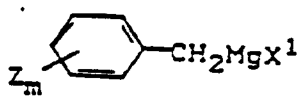

(VI)

dans laquelle

Z et m ont la signification mentionnée ci-dessus

et

$X^1$ représente un atome de chlore, un atome de brome ou un atome d'iode,

en présence d'un diluant

ou

d) des dérivés d'azolylméthyl-cyclopropylcarbinol de formule

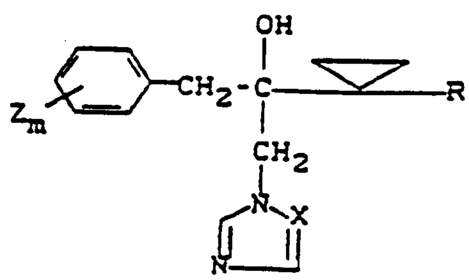

(Ia)

dans laquelle

R, X, Z et m ont la signification mentionnée ci-dessus,

avec des bases fortes en présence d'un diluant, et en faisant réagir les alcoolates que l'on obtient en l'occurrence et répondant à la formule

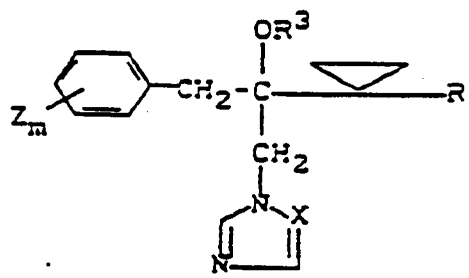

(Ib)

dans laquelle

R, X, Z et m ont la signification mentionnée ci-dessus et

$R^3$ représente un cation de métal alcalin ou un cation de phosphonium ou d'ammonium quaternaire,

avec des composés halogénés de formule

$R^4$-Hal' (VII)

où

$R^4$ représente un groupe alkyle contenant de 1 à 6 atomes de carbone ou un groupe alkylcarbonyle contenant de 1 à 6 atomes de carbone dans la fraction alkyle et

Hal' représente un atome de chlore ou un atome de brome,

en présence d'un diluant

et ensuite, on ajoute éventuellement un acide ou un sel métallique aux composés de formule (I) ainsi obtenus.

2. Fongicides et agents régulateurs de la croissance des plantes, **caractérisés par** une teneur en au moins un dérivé d'azolylméthyl-cyclopropyle de formule (I) selon la revendication 1 ou en un sel d'addition d'acide ou encore d'un complexe de sel métallique d'un dérivé d'azolylméthylcyclopropyle de formule (I).

3. Utilisation de dérivés d'azolylméthylcyclopropyle de formule (I) selon la revendication 1 ou de leurs sels d'addition d'acides ou encore de leurs complexes de sels métalliques, pour lutter contre les champignons, ainsi que pour régulariser la croissance des plantes.

4. Procédé pour la préparation de dérivés de propanol de formule

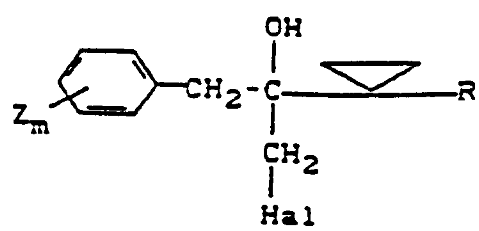

(II)

dans laquelle

R, Z, Hal et m ont les significations mentionnées ci-dessus,

**caractérisé en ce qu'**on fait réagir des cyclopropyl-cétones de formule

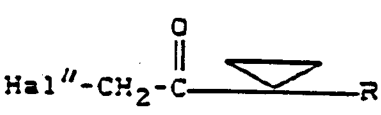

(VIII)

dans laquelle

R a la signification mentionnée ci-dessus et

Hal" représente un atome de chlore ou un atome de brome

avec des composés organométalliques de formule

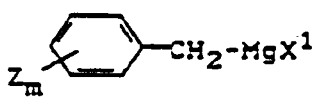

(VI)

dans laquelle

Z et m ont la signification mentionnée ci-dessus et

$X^1$ représente un atome de chlore, un atome de brome ou un atome d'iode, en présence d'un diluant.

**5.** Procédé pour la préparation d'oxirannes de formule

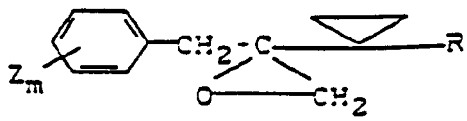

(IV)

dans laquelle

R, Z et m ont les significations mentionnées ci-dessus,

**caractérisé en ce qu'**on fait réagir des dérivés de propanol de formule

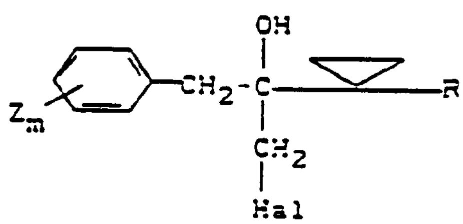

(II)

dans laquelle

R, Z et m ont la signification mentionnée ci-dessus et

Hal représente un atome de chlore, un atome de brome ou un atome d'iode, avec des bases en présence d'un diluant.

**6.** Procédé pour la préparation d'azolylméthylcétones de formule

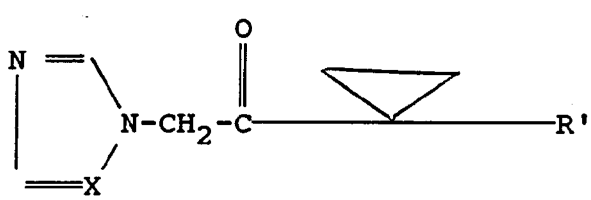

(V-a)

dans laquelle

R' représente un atome de fluor, un atome de chlore, un atome de brome, un groupe phényle éventuellement substitué 1 à 3 fois de manière identique ou différente par un atome de fluor, un atome de chlore, un atome de brome, un groupe trifluorométhyle, un groupe méthyle, un groupe éthyle, un groupe méthoxy et/ou un groupe éthoxy, ou représente le groupement -Y-$R^{2'}$ où

Y représente un atome d'oxygène, un atome de soufre, SO ou $SO_2$, et

$R^{2'}$ représente un groupe phényle éventuellement substitué 1 à 3 fois de manière identique ou différente par un atome de fluor, un atome de chlore, un atome de brome, un groupe trifluorométhyle, un groupe méthyle, un groupe éthyle, un groupe méthoxy et/ou un groupe éthoxy, et

X représente un atome d'azote ou un groupe CH.

**caractérisé en ce qu'**on fait réagir des cyclopropylcétones de formule

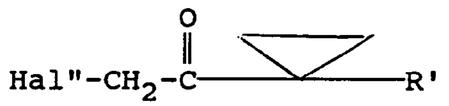

(VIII-a)

dans laquelle

R' a la signification mentionnée ci-dessus et

Hal" représente un atome de chlore ou un atome de brome

avec des azoles de formule

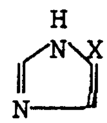

(III)

dans laquelle

X a la signification mentionnée ci-dessus,

en présence d'un agent fixateur d'acides et éventuellement en présence d'un diluant.